(19) Europäisches Patentamt
European Patent Office
Office européen des brevets

(11) **EP 1 928 511 B1**

(12) **EUROPEAN PATENT SPECIFICATION**

(45) Date of publication and mention
of the grant of the patent:
**19.01.2011 Bulletin 2011/03**

(51) Int Cl.:
***A61L 15/60*** (2006.01)     ***A61F 13/15*** (2006.01)

(21) Application number: **06804128.4**

(22) Date of filing: **25.09.2006**

(86) International application number:
**PCT/US2006/037320**

(87) International publication number:
**WO 2007/041075 (12.04.2007 Gazette 2007/15)**

(54) **ABSORBENT ARTICLE COMPRISING A PRIMARY AQUEOUS-LIQUID-ABSORBING AGENT**

SAUGFÄHIGER ARTIKEL MIT EINEM ERSTEN WÄSSRIG-FLÜSSIG-SAUGFÄHIGEM WIRKSTOFF

ARTICLE ABSORBANT COMPRENANT UN AGENT ABSORBANT DE LIQUIDE AQUEUX PRIMAIRE

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.09.2005 JP 2005289398**

(43) Date of publication of application:
**11.06.2008 Bulletin 2008/24**

(73) Proprietor: **The Procter and Gamble Company Cincinnati, OH 45202 (US)**

(72) Inventors:
• **IKEUCHI, Hiroyuki**
**Himeji-shi, Hyogo 670-0001 (JP)**
• **SAKAMOTO, Shigeru**
**Himeji-shi, Hyogo 670-0001 (JP)**
• **MACHIDA, Sayaka**
**Himeji-shi, Hyogo 670-0001 (JP)**
• **BERUDA, Holger**
**65834 Schwalbach (DE)**
• **NAKAGAWA, Yasue**
**81927 München (DE)**

(74) Representative: **McGregor, Judit Ester et al Procter & Gamble Service GmbH Sulzbacher Strasse 40-50 65824 Schwalbach am Taunus (DE)**

(56) References cited:
**EP-A2- 1 516 884**     **WO-A-03/043670**
**WO-A-2005/044915**     **WO-A-2005/097313**

**Description**

FIELD OF THE INVENTION

The present invention relates to an absorbent article, such as a diaper or adult incontinence article or sanitary napkin, comprising a primary aqueous-liquid-absorbing agent comprising resin particles obtained by a process including the step of polymerizing a water-soluble ethylenically unsaturated monomer having a carboxyl group, in the presence of an internal-crosslinking agent having at least four functional groups each capable of forming a covalent bond with a carboxyl group, the aqueous-liquid-absorbing agent being characterized by exhibiting a water absorption capacity (CRC) of 5 to 25 g/g and a saline flow conductivity (SFC) of not less than $1216 \times 10^{-7}$ cm$^3$ s/g.

BACKGROUND TO THE INVENTION

There have been continuous investigations to provide improved absorbent article with so-called super-absorbing resins or material or water-absorbing material or water-gelling material Such materials are often surface-cross-linked or internally cross-linked. Ep1516884. WO05/04491 and WO05/097313 describe suitable surface-cross-linking agent or internal-cross-linking agents. Typically such articles need to that have a high absorption rate, absorption amount, and retention for aqueous liquids and therefore the prior art water-absorbing resins are typically being mixed with or used in addition to fibrous materials such as cellulose fiber, polyester fiber, polyethylene fiber, and/or polypropylene fiber.

In recent years, the ratios of the water-absorbing resins to fibrous material in the absorbent articles tend to increase with the increasing needs of the thinning of the sanitary materials such as diapers. For realization of thinner sanitary materials, it is desired that the fiber materials are replaced with water-absorbent resins for further increase in ratio of the water-absorbent resins in the absorbent structures.

The water-absorbent resin is inherently excellent in the performances of absorbing and retaining the aqueous liquid. However, the fibrous materials are poor in these performances, particularly the performance of retaining the aqueous liquid, and have different performances from those of the conventional water-absorbent resin. As such, as + a future water-absorbent resin that responds to the needs, water-absorbent resins having the performances of the fibrous materials in the conventional absorbent structures must be developed. Examples of the performances demanded to such a water-absorbent resin having the performances of the fibrous materials include: a performance of rapidly absorbing an aqueous liquid; a performance of diffusing the aqueous liquid after having absorbed it; and a performance of being capable of temporarily retaining the aqueous liquid after having absorbed it. Therefore, the development of water-absorbent resins having these performances has been desired.

The present invention provides absorbent articles comprising such an improved water-absorbent resin or aqueous-liquid-absorbing agent, which can be made much thinner or more flexible than conventional absorbent articles, because the aqueous-liquid-absorbing agent has the performances of traditionally used fibrous materials i.e. the performance of rapidly acquiring an aqueous liquid; the performance of diffusing the aqueous liquid after having acquired it; and the performance of being capable of temporarily retaining the aqueous liquid after having acquired it.

SUMMARY OF THE INVENTION

The present invention relates to an absorbent article comprising an absorbent core, which comprises a primary aqueous-liquid-absorbing agent that contains water-absorbent resin particles, wherein the water-absorbent resin particles are obtained by a process including the step of polymerizing a water-soluble ethylenically unsaturated monomer having a carboxyl group, said resin particles being internally cross-linked with the selected internal-cress-linking agent described herein, and surface-crosslinked, whereby the primary aqueous-liquid-absorbing agent exhibits a water absorption capacity (CRC) of 5 to 25 g/g and a saline flow conductivity (SFC) of not less than $1216 \times 10^{-7}$ cm$^3$ s/g, and preferably having an absorption rate (FSR) of not less than 0.1 g/g/s and/ or preferably having a wet porosity of not less than 20 %.

The absorbent article is preferably a disposable absorbent article, preferably selected from sanitary napkins, panty-liners, adult incontinence articles and diapers, including training or pull-up pants.

The absorbent article preferably comprises a topsheet, backsheet and therein between an absorbent core, that may comprise one or more acquisition storage layers, one or more being in contact with the topsheet, and there underneath one or more storage layers, whereby at least one of the layers comprises the primary aqueous-liquid-absorbing agent herein, said layer(s) comprising preferably less than 10% by weight (of the primary aqueous-liquid-absorbing agent in said layer) of cellulose fibers (including modified cellulose fibers such as chemically or mechanically modified cellulose fibers).

The absorbent core preferably also comprises a secondary aqueous-liquid absorbing agent, having a SFC of less than $600 \times 10^{-7}$ cm$^3$ s/g, which may be comprised in the same or a different layer of the absorbent core to the primary aqueous-liquid- absorbing agent, whereby one or more or all of the layers of the core are preferably substantially free of cellulose-type fibers, e.g. comprising each less than 10% by weight (by weight of all (i.e. primary or secondary) liquid-absorbing agents present in said layer) of cellulose fibers.

The disposable absorbent article herein are preferably very thin, having preferably a maximum dry caliper in the crotch region (as measured herein) of 4.5 mm or less.

The disposable absorbent articles herein comprise preferably an absorbent core comprising at least said primary aqueous-liquid-absorbing agent and optionally said secondary aqueous-liquid-absorbing agent, and said core having one or more regions with an average density greater than about 0.2 g/cm$^3$, or said core as a whole having such a density.

FIGURES

Fig. 1: A schematic sectional view of a measurement apparatus as used for measuring the AAP.

Fig. 2: A schematic sectional view of a measurement apparatus as used for measuring the SFC.

Fig. 3: A schematic sectional view of a portion of the measurement apparatus as used for measuring the SFC.

Fig. 4: A bottom view of a piston head of the measurement apparatus as used for measuring the SFC.

DEATAILED DESCRIPTION

Absorbent articles

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers (adult and infant; including training pants), adult incontinence briefs, diaper holders and liners, sanitary napkins, panty-liners and tampons.

"Diaper" refers to an absorbent article generally worn by infants and incontinent persons about the lower torso.

"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).

"Region", when used herein, as a region of a absorbent core or layer thereof, means herein an area (with when applicable a z-direction calliper) of 2 cm x 2 cm.

The absorbent articles envisaged herein comprise a supporting structure and the aqueous-liquid-absorbing agent described herein.

A preferred absorbent article herein preferably comprises a topsheet and a backsheet, described herein below, with therein between an absorbent core. A preferred article herein is a diaper or adult incontinent brief or sanitary napkin, which comprises a chassis, which comprises an outer covering including a liquid pervious topsheet and backsheet. The chassis preferably further includes side panels, (elasticized) leg cuffs and/ barrier cuffs, and (elastic) waist feature. One end portion of the article is configured as a first waist region of the article. The opposite end portion is configured as a second waist region. An intermediate portion is configured as a crotch region, which extends longitudinally between the first and second waist regions. The crotch region is that portion which, when the article such as a diaper or adult incontinence brief or sanitary napkin is worn, is generally positioned between the wearer's legs. The chassis may also comprise a fastening system, which may include at least one fastening member and at least one landing zone.

For unitary absorbent articles like diapers, the chassis comprises the main structure of the diaper with other features added to form the composite diaper structure. While diaper may be assembled in a variety of well-known configurations, preferred diaper configurations are described generally in US Pats. 4,940,464, 5,554,145; 5,569,234; 6,004,306, U.S. Pat. Application Serial No. 10/171,249 and in U.S. Pat. Application Serial No. 10/824,121.

The topsheet is compliant, soft feeling, and non-irritating to the wearer's skin. It may be liquid pervious, permitting liquids to readily penetrate through its thickness. A suitable topsheet can be manufactured from a wide range of materials such as porous foams, reticulated foams, apertured plastic films, natural fibers (e.g., wood or cotton fibers), synthetic fibers (e.g., polyester or polypropylene fibers) or from a combination of natural and synthetic fibers. In one embodiment, the topsheet is made of a hydrophobic material to isolate the wearer's skin from liquids in the absorbent core, and it may then comprise one or more opening to receive the bodily exudates. Preferably the topsheet comprises a means to adjust hydrophilicity of the material, like a surfactant. A preferred topsheet comprises a nonwoven material made using means well known to those skilled in the fabrics art. Preferably, the topsheet has a basis weight from about 10 to about 25 g/m$^2$,

a minimum dry tensile strength of at least about 150 g/cm in the machine direction and a strikethrough of less than about 3 seconds according to European Disposables and Nonwovens Association standard method 150.4-99. One suitable topsheet comprises a polypropylene spunbonded nonwoven comprises fibers of less than 3 denier having a basis weight of about 18 g/m$^2$ as is available from BBA Fiberweb of Simpsonville, SC.

The backsheet is preferably joined to the topsheet at least about a portion of the periphery thereof. The backsheet prevents exudates absorbed by the absorbent core and contained within the article from soiling other external articles that may contact the article, such as bed sheets and clothing. The backsheet is preferably manufactured from a thin polymer film. In one preferred embodiment the film is impervious to liquids. Typically, the backsheet comprises a layer of polyethylene film having a basis weight between about 10 g/m$^2$ and about 30 g/m$^2$, although other flexible, liquid impervious materials can be used. Preferably, the film is breathable (e.g., via micropores) so as to permit vapors to escape from the diaper while still preventing exudates from passing through the backsheet. Particularly preferred backsheet materials have a nonwoven laminated to the film layer so as to make backsheet more "cloth-like". Such a nonwoven layer may comprise a nonwoven material (e.g., one having a spunbonded or other suitable structure) with a basis weight between about 15 g/m$^2$ and about 25 g/m$^2$. Suitable materials for use as backsheet are available form Clopay Plastic Products Company of Mason, OH. Additional features for absorbent articles are well known in the art and are e.g., described in US Pat. 3,860,003 and US Pat. 5,151,092.

The absorbent article herein typically comprises an absorbent core or layer that comprises the primary aqueous-liquid-absorbing agent described herein. Exemplary absorbent structures or cores for use herein are described in US Pats. 4,610,678; 4,834,735; 5,260,345; 5,387,207; 5,397,316; and 5,625,222.

The absorbent core may comprise one or more than one layers, for example a first acquisition/ storage layer, preferably in close contact with the topsheet, and a storage layer, as described herein below in more detail. The absorbent core may comprises also an acquisition layer underlying the topsheet and then the acquisition/storage layer, mentioned above, disposed between the acquisition layer and the remaining component of core, e.g. said storage layer.

Such an acquisition/storage layer provides acquisition along with temporary distribution and storage of acquired fluids and optionally permanent storage of a portion thereof and said storage layer provides the majority (e.g. more than 60%) of the storage capacity of the article.

The absorbent core comprises the primary aqueous-liquid-absorbing agent described herein, and it may comprise in addition a secondary aqueous-liquid-absorbing agent with a SFC of less than 600 units as described herein in more detail.

The absorbent core comprises preferably very little cellulose fibres, preferably less than 10% by weight of the total of primary and if present secondary aqueous-liquid absorbing agent.

Thus, an acquisition/ storage layer or storage layer or absorbent core that is "substantially cellulose free" means that the structure comprises less than 10% (by total weight of all of the aqueous-liquid absorbing agent or agents, i.e. primary and/ or secondary liquid absorbing agent, present in said layer or core; more preferably this level is less than 5% or less than 1%, or no cellulose fibres at all; typically it means that the layer (s) or core comprises at least about 90% by weight of the layer or core of aqueous-liquid-absorbing agent or agents, e.g. said primary and/or said secondary aqueous-liquid-absorbing agent.

As mentioned above, the absorbent articles herein preferably comprise a secondary aqueous-liquid-absorbing agent that, in contrast to the primary aqueous-liquid-absorbing agent, has a SFC of less than 600 x 10$^{-7}$ cm$^3$ s/g, preferably less than 400 x10$^{-7}$cm$^3$s/g or even preferably less than 200 x10$^{-7}$cm$^3$s/g, and typically at least 40 x10$^{-7}$cm$^3$s/g, or preferably at least 80 x10$^{-7}$ cm$^3$.s/g, or preferably at least 100 x 10$^{-7}$cm$^3$s/g or even at least 120 x 10$^{-7}$cm$^3$s/g. The secondary aqueous-liquid absorbing agent typically has a CRC of at least 20 g/g, preferably at least 25 g/g or preferably at least 30 g/g or even preferably at least 35 g/g.

The secondary aqueous-liquid absorbing agent may be present at a higher level by weight than the primary aqueous-liquid absorbing agent.

The secondary aqueous-liquid absorbing agent may be mixed with the primary liquid absorbing agent, or it may be present in a separate region or layer; typically it is al least present in a so-called storage layer, described herein.

The primary aqueous-liquid absorbing agent described herein is preferably comprised by at least an acquisition/ storage layer of the absorbent article, as described herein.

In particularly preferred embodiments, the absorbent core is narrower in crotch region than it is in either of the waist regions. Preferably, the ratio of the width of core at transverse axis to the widest lateral width thereof in either of first waist region or second waist region is less than 0.8, or more preferably less than about 0.7.

The absorbent article may comprise a, preferably thin, e.g. less than 1 mm thick on average, cellulose containing acquisition layer, such as chemically stiffened, curled and/ or twisted cellulose. In one embodiment, the absorbent article does not comprise any acquisition layer, or acquisition/ storage layer and/ or storage layer comprising more than 10% (by weight of such layer) of cellulose fibres, as mentioned above.

The acquisition/storage layer, which may be in contact with the topsheet or an acquisition layer, may be in direct contact with a storage layer, or an intermediate layer may be present, such as a nonwoven storage layer cover material. The acquisition/ storage layer and/ or storage layer may be covered completely or partially by one or more of such cover materials. One preferred cover material comprises a spunbonded, a melt-blown and a further spunbonded layer (i.e., a SMS material). The non-woven materials are suitably made using synthetic fibers, such as polyethylene, polyester and, most preferably, polypropylene. Highly preferred are permanently hydrophilic non-wovens, and in particular nonwovens with durably hydrophilic coatings. Such hydrophilicity may be provided by surfactant treatment of the non-woven. An alternative material comprises an SMMS-structure or a cellulosic tissue structure. The cover material may also be substantially free of cellulose fibers.

Suitably, the acquisition/storage layer has the same surface area as the storage layer or smaller. Preferably, the acquisition/storage layer is laterally centred on the storage layer with the same lateral width but a shorter longitudinal length than storage system. The acquisition/storage layer may also be narrower than the storage layer while remaining centred thereon. Said another way, the acquisition/storage layer may suitably have an area ratio with respect to the storage system of 1.0. Preferably, the area ratio is less than 1.0 (e.g., less than about 0.75), more preferably less than about 0.50.

The acquisition/storage layer and/ or storage layer may comprise each an aqueous-liquid-absorbing agent, typically each comprise a different, i.e. a primary or secondary, aqueous-liquid-absorbing agent and/ or a different mixture of two or more different aqueous-liquid-absorbing agents. They may comprise an uneven distribution of the aqueous-liquid-absorbing agent(s) basis weight in one or both of the machine and cross directions.

In one embodiment, the absorbent core, the acquisition/ storage layer and/ or the storage layer thereof is obtained by stabilising the primary and/ or secondary aqueous-liquid-absorbing agent with a fibrous layer of thermoplastic (adhesive) material, e.g. which allows removal of some or all of the (absorbent) cellulose fibres that are often present in the storage layer or core to stabilise the aqueous-liquid-absorbing, agent(s). The core, storage layer and/ or acquisition/ storage layer may be substantially cellulose (fibres) free, as mentioned above and as is for example also described in aforementioned US Pat. Application Serial No. 10/776,839.

The primary or typically secondary aqueous-liquid-absorbing agent of the storage layer may have an average basis weight of at least about 200 g/m$^2$, or at least at 300g/m$^2$. The storage layer may have one or more regions with an average density greater than about 0.2 g/ cm$^3$, or preferably at least 0.3 g/cm$^3$, or possibly even greater than 0.4 g/ cm$^3$.

Because an acquisition/storage layer may be substantially free of cellulosic fibres, it may have a higher density than components of an absorbent core used by the prior art for similar purposes. Suitably, an acquisition/storage layer, comprising the primary aqueous-liquid-absorbing agent herein, may have one or more regions with an average density greater 0.2 g/ cm$^3$ or more, preferably 0.3 g/cm$^3$ or more, or possibly even greater than 0.4 g/ cm$^3$.

The absorbent article herein is preferably very thin, having an absorbent core with a maximum dry calliper in the crotch region of less than about 4.5 mm, preferably less than 4.0 mm, or even preferably less than 3.5mm, or even less than 3.0 mm. This is measured by dividing the crotch regions in areas of 2 cm x 2 cm, and measuring the calliper per region. The absorbent article preferably is also very thin, having preferably a maximum dry caliper of less than about 5.5 mm, preferably less than 5.0 mm, or even preferably less than 4.5mm, or even less than 4.0, as measured in the crotch region as set out above.

A suitable storage layer may be produced using the method described in the aforementioned US Pat Application Serial No. 10/776,839; a suitable acquisition/ storage layer may be produced as described in US Pat. Application Serial No. 10/776,839, a laydown drum is provided with a series of "pockets" having a shape and volume substantially defined by the desired shape and volume of acquisition/storage layer.

Primary aqueous-liquid-absorbing agent

The absorbent articles of the invention comprise at least a primary aqueous-liquid-absorbing agent exhibiting a water absorption capacity (CRC) of 5 to 25 g/g and a saline flow conductivity (SFC) of not less than 1216 x 10$^{-7}$ cm$^3$ s/g, as claimed herein, and as is measured the CRC and SFC method described herein, said agent typically obtainable by a process comprising the steps of: (a) polymerizing a water-soluble ethylenically unsaturated monomer having a carboxyl group in an aqueous monomer solution including the water-soluble ethylenically unsaturated monomer having a carboxyl group, in the presence of an internal-crosslinking agent having at least four functional groups each capable of forming a covalent bond with a carboxyl group to thereby obtain a hydropolymer; (b) drying the hydropolymer obtained in the step (a) at a temperature of not less than 150°C to thereby obtain water-absorbent resin particles; and (c) surface-crosslinking the water-absorbent resin particles obtained in the step (b), wherein the amount (Y) (mol %) of the internal-crosslinking agent as used, relative to the water-soluble ethylenically unsaturated monomer having a carboxyl group, is expressed by the following equation (1): $Y \geq 0.06/\{2-(2.35X/100)\}$ ...(1) where X is neutralization degree (mol %) of a carboxyl group in the water-absorbent resin particles and is in the range of 45 to 85 mol %.

The process for producing a primary aqueous-liquid-absorbing agent of the articles of the present invention, favorably further comprises the step of pulverizing the obtained hydropolymer or the obtained water-absorbent resin particles at least before or after the step (b) and/ or an agglomeration step, because the water-absorbent resin particles are preferably agglomerate particles.

In the present invention, the water-absorbent resin refers to a hydrogel-formable, water-swellable, and water-insoluble crosslinked polymer.

The CRC upper limit value is preferably 18 g/g, or16 g/g, especially or 14 g/g, or 12 g/g. The CRC lower limit value is 5 g/g, more preferably 9 g/g, still more favorably 10 g/g.

The SFC as measured by the method herein is preferably not less than $1300 \times 10^{-7}$ $cm^3$ s/g, or not less than $1400 \times 10^{-7}$ $cm^3$ s/g, or not less than $1450 \times 10^{-7}$ $cm^3$ s/g, or not less than $1500 \times 10^{-7}$ $cm^3$ s/g, or not less than $1600 \times 10^{-7}$ $cm^3$ s/g. Its upper limit value is not particularly limited, but the upper limit value is favorably not more than $4000 \times 10^{-7} cm^3$ s/g.

The primary aqueous-liquid-absorbing agent herein favorably exhibits an absorption rate (modified FSR) of not less than 0.1 g/g/s, more favorably not less than 0.15 g/g/s, still more favorably not less than 0.2 g/g/s, especially favorably not less than 0.25 g/g/s, most favorably not less than 0.3 g/g/s. Its upper limit value is not particularly limited, but is favorably not more than 5 g/g/s, more favorably not more than 2 g/g/s, still more favorably not more than 1 g/g/s.

The primary aqueous-liquid-absorbing agent herein favorably exhibits a wet porosity of not less than 20 %, more favorably not less than 30 %, still more favorably not less than 35 %, especially favorably not less than 40 %. Its upper limit value is not particularly limited, but is favorably not more than 60 %, more favorably not more than 50 %.

The primary aqueous-liquid-absorbing agent herein may exhibit absorbency against pressure (AAP) in the range of favorably 5 to 25 g/g, more favorably 11 to 22 g/g.

The primary aqueous-liquid-absorbing agent herein may have an extractable component content in the range of favorably 0 to 15 weight %, more favorably 0 to 10 weight %, still more favorably 0 to 8 weight %.

The primary aqueous-liquid-absorbing agent may be made by a process including the step of subjecting the water-absorbent resin particles to treatment for liquid permeability enhancement before or after the step (c) above. The treatment for liquid permeability enhancement may be carried out by adding a liquid-permeability-enhancing agent. The liquid-permeability-enhancing agent is favorably at least one member selected from among water-soluble multivalent metal compounds and water-soluble polycationic compounds.

The primary aqueous-liquid-absorbing agent of the articles herein is an aqueous-liquid-absorbing agent containing water-absorbent resin particles as essential components being internally crosslinked and being surface-crosslinked, and comprising polymerized water-soluble ethylenically unsaturated monomers, the aqueous-liquid-absorbing agent being characterized by exhibiting a water absorption capacity (CRC) of 5 to 25 g/g and a saline flow conductivity (SFC ) of not less than $1216 \times 10^{-7}$ $cm^3$ s/g

Preferably, the absorbent article also comprises a secondary aqueous-liquid-absorbing agent, having a different performance, e.g. and suitable a different chemistry, as described above. This agent may be made by the method described herein, but typically having different crosslinking levels, surface-crosslinking levels and/ or different chemistry, e.g. of the cross-linking compounds, compared to the primary aqueous-liquid-absorbing agent; such secondary aqueous-liquid absorbing agents as used herein are known in the art.

The primary aqueous-liquid-absorbing agent herein has preferably an absorption rate (modified FSR) of not less than 0.1 g/g/s, and on the other hand, that, in order for the above primary aqueous-liquid-absorbing agent to have the aqueous-liquid-retaining ability capable of further temporarily retaining the aqueous-liquid after having quickly absorbed it, the above aqueous-liquid-absorbing agent favorably exhibit a wet porosity of not less than 20 %, the wet porosity being an index of proportion of the volume of pore in a gel layer formed in an aqueous-liquid-absorbing agent swollen (wetted) under load, relative to the volume of the gel layer.

Note that as the wet porosity is higher, the primary aqueous-liquid-absorbing agent is able to acquire the aqueous-liquid at one go, retain the aqueous-liquid temporarily quickly, and then diffuse the temporally retained aqueous-liquid because of a large pore there between. Also, it can be said that the wet porosity is an index of the amount of liquid that can be retained between gels other than the aqueous-liquid which is absorbed by the water absorbent resin and exists in the gel. Therefore, as the wet porosity is higher, the primary aqueous-liquid-absorbing agent can retain still more aqueous-liquid there between, in addition to the liquid having been absorbed therein, after having acquired the aqueous-liquid.

The "water-absorbent resin particles as the main components" herein refer to 50 weight % or more water-absorbent resin particles content relative to the entire primary aqueous-liquid-absorbing agent. The water-absorbent resin particles content is favorably in the range of 60 to 100 weight %, more favorably 70 to 100 weight %, still more favorably 80 to 100 weight %, yet more favorably 90 to 100 weight %, relative to the entire aqueous-liquid-absorbing agent.

The primary aqueous-liquid-absorbing agent comprises preferably only low levels or no uncrosslinked extractable component, e.g. 0 to 50 weight %, more favorably not more than 25 weight %, still more favorably not higher than 20 weight % by weight of the water-absorbent resin.

Specific examples of the hydrogel-formable, water-swellable, and water-insoluble crosslinked polymer or its particles include: partially-neutralized and crosslinked polymers of poly(acrylic acids) (e.g. USP 4,625,001, USP 4,654,039, USP 5,250,640, USP 5,275,773, EP 0456136); crosslinked and partially-neutralized graft polymers of starch-acrylic acid (USP 4,076,663); copolymers of isobutylene-maleic acid (USP 4,389,513); saponified copolymers of vinyl acetate-acrylic acid (USP 4,124,748); hydrolyzed (co)polymers of acrylamide (USP 3,959,569); and hydrolyzed polymers of acrylonitrile (USP 3,935,099).

The water-absorbent resin particles that can be used herein are favorably of 100% particulate shape. Examples of the particulate shape includes: a spherical shape; a shape of an agglomerate of spheres; a shape like a flattened sphere; an irregularly pulverized shape; a shape of an agglomerate of irregularly pulverized materials; and a foamed shape having pores. Incidentally, in the present invention, the water-absorbent resin particles may be referred to simply as water-absorbent resin.

In the case where the primary aqueous-liquid-absorbing agent used in the present invention is particulate, the particle diameters and particle diameter distribution of this agent are free of especial limitation. However, for still more exerting the effects of the present invention, it is favorable that the weight-average particle diameter of this agent is in the range of 150 to 850 $\mu$m, more favorably 150 to 600 $\mu$m, more favorably 150 to 500 $\mu$m, more favorably 200 to 400 $\mu$m, still more favorably 250 to 380 $\mu$m, and also it is favorable that the logarithmic standard deviation ($\sigma\zeta$) of this agent is favorably in the range of 0.1 to 0.45, more favorably 0.2 to 0.45, still more favorably 0.25 to 0.40, yet more favorably 0.30 to 0.35. Examples of the water-absorbent resin particles and aqueous-liquid-absorbing agent herein having a favorable combination of weight-average particle diameter (D50) and logarithmic standard deviation ($\sigma\zeta$) of particle diameter distribution includes: those having a weight-average particle diameter (D50) of not less than 200 $\mu$m and less than 400 $\mu$m, and a logarithmic standard deviation ($\sigma\zeta$) of particle diameter distribution of not less than 0.20 and not more than 0.45 (those having a small average particle diameter and narrow particle diameter distribution); and those having a weight-average particle diameter (D50) of not less than 400 $\mu$m and not more than 750 $\mu$m, and a logarithmic standard deviation ($\sigma\zeta$) of particle diameter distribution of not less than 0.20 and not more than 0.45 (those having a large average particle diameter and narrow particle diameter distribution).

It may be preferred that this agent includes particles having particle diameters in the range of 150 to 850 $\mu$m in an amount of not smaller than 90 to 100 weight %. Further, it is more favorable that this agent includes particles having particle diameters in the range of 150 to 600 $\mu$m in an amount of not smaller than 90 to 100 weight %, particularly favorably not smaller than 95 to 100 weight %. Further, it is more favorable that this agent includes particles having particle diameters in the range of 150 to 500 $\mu$m in an amount of not smaller than 90 to 100 weight %, particularly favorably not smaller than 95 to 100 weight %.

The water-absorbent resin particles for the primary and optionally also the secondary aqueous-liquid absorbing agent herein are obtained by a process including the step of polymerizing a water-soluble ethylenically unsaturated monomer having a carboxyl group, and having a crosslinked structure.

Examples of water-absorbent resin particles obtained by a process including the step of polymerizing a water-soluble ethylenically unsaturated monomer having a carboxyl group include: polymers obtained by polymerizing and crosslinking carboxyl-group containing unsaturated monomers having a carboxyl group such as (meth)acrylic acid, maleic anhydride, maleic acid, fumaric acid, crotonic acid, itaconic acid, and cinnamic acid, and/or their salts (neutralizer); hydrolyzed graft polymers of starch-acrylonitrile; graft polymers of starch-acrylic acid; saponified copolymers of vinyl acetate-acrylate acid; hydrolyzed or crosslinked acrylonitrile copolymers or acrylamide copolymers; denatured carboxyl-group containing crosslinked polyvinyl alcohol; crosslinked copolymers of isobutylene-maleic anhydride; or either one or a combination of two or more substances among the above substance. Above all, the water-absorbent resin particles are water-absorbent resin particles including a crosslinked polyacrylic acid (salt) polymer obtained by a process including the step of polymerizing a monomer including acrylic acid and/or its salt as a main component.

The crosslinked polyacrylic acid (salt) polymer useful herein is a polymer which is obtained by a process including the step of polymerizing a monomer (excluding cross-linking agent) containing acrylic acid and/or its salt in an amount of favorably 50 to 100 mol %, more favorably 70 to 100 mol %, still more favorably 90 to 100 mol %, and has a crosslinked structure in its inside. In addition, 45 to 85 mol % of the carboxyl group in the water-absorbent resin particles are favorably neutralized to form salt. In other words, the carboxyl group of the water-absorbent resin particles preferably has a neutralization degree of 45 to 85 mol %, more favorably 50 to 85 mol %, still more favorably 55 to 80 mol %, especially favorably 60 to 75 mol %. As examples of the salt, there can be cited at least one of such as: alkaline metal (e.g. sodium, potassium, lithium) salts, ammonium salts, and amine salts. The neutralization of the carboxyl group for forming the salt may be carried out in a monomer state before the polymerization, or may be carried out in a polymer state on the way of or after the polymerization, or may be carried out both in these states.

Incidentally, a neutralization degree of a carboxyl group in the water-absorbent resin particles can be obtained by calculation from (i) the amount of water-soluble ethylenically unsaturated monomer having a not-yet-neutralized carboxyl group and (ii) the total amount of bases as used for neutralization before the polymerization, during the polymerization, and/or after the polymerization. Alternatively, as mentioned below, the neutralization degree may be obtained by titration of extractable component content in the water-absorbent resin particles.

The water-absorbent resin particles favorably used in the present invention may, if necessary, realized by a copolymer obtained by copolymerizing another monomer jointly with the water-soluble ethylenically unsaturated monomer having a carboxyl group (if crosslinked polyacrylic acid (salt) polymer, acrylic acid and/or its salt) used as the main component.

Specific examples of the above other monomer include: anionic unsaturated monomers (e.g. methacrylic acid, maleic acid, vinylsulfonic acid, styrenesulfonic acid, 2-(meth)acrylamido-2-methylpropanesulfonic acid, 2-(meth)acryloylethanesulfonic acid, 2-(meth)acryloylpropanesulfonic acid) and their salts; nonionic-hydrophilic-group-containing unsaturated monomers (e.g. acrylamide, methacrylamide, N-ethyl(meth)acrylamide, N-n-propyl(meth)acrylamide, N-isopropyl(meth)acrylamide, N,N-dimethyl(meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinylpyridine, N-vinylpyrrolidone, N-acryloylpiperidine, N-acryloylpyrrolidine, N-vinylacetamide); and cationic unsaturated monomers (e.g. N,N-dimethylaminoethyl (meth)acrylate, N,N-diethylaminoethyl (meth)acrylate, N,N-dimethylaminopropyl (meth)acrylate, N,N-dimethylaminopropyl(meth)acrylamide, and their quaternary salts). The amount of these monomers used as monomers other than acrylic acid and/or its salt is favorably in the range of 0 to 30 mol %, more favorably 0 to 10 mol %, of the entire monomers.

The water-absorbent resin particles, usable in the present invention, have a crosslinked structure in their inside and surface, i.e. they are internally cross-linked. As to methods for introducing the internal crosslinked structure into the water-absorbent resin particles as used in the present invention, examples thereof include: a method in which the introduction is carried out by self-crosslinking without any crosslinking agent; and a method in which the introduction is carried out by copolymerization or reaction with an internal-crosslinking agent having at least two polymerizable ethylenical double bonds and/or at least two functional groups per molecule. The functional groups are highly reactive groups in a molecule and includes a covalent-bondable functional group and an ion-bondable functional group. For still more exerting the effects of the present invention, it is favorable that the water-absorbent resin particles usable in the present invention are surface-crosslinked ones.

The primary aqueous-liquid-absorbing agent herein may contain small amount of additive and/or water, if necessary. The primary aqueous-liquid-absorbing agent of the present invention favorably contains a liquid-permeability-enhancing agent as the additive. Especially, in the case where the primary aqueous-liquid-absorbing agent includes water-absorbent resin particles not subjected to treatment for liquid permeability enhancement as main components, it is very favorable that it further includes a liquid-permeability-enhancing agent. On the other hand, in the case where the primary aqueous-liquid-absorbing agent includes water-absorbent resin particles subjected to treatment for liquid permeability enhancement, the primary aqueous-liquid-absorbing agent according to the present invention may be realized by only the water-absorbent resin particles. The liquid-permeability-enhancing agent herein refers to an agent that enhances the SFC.

Examples of the liquid-permeability-enhancing agent include: water-soluble multivalent metal compounds (e.g. aluminum sulfate, potassium alum, ammonium alum, sodium alum, (poly)aluminum chloride, and their hydrates); water-soluble polycationic compounds (e.g. polyethylenimine, polyvinylamine, polyallylamine); and water-insoluble inorganic fine particles (e.g. silica, alumina, bentonite). These may be used either alone respectively or in combinations with each other. Above all, water-soluble multivalent metal salts (e.g. aluminum sulfate, potassium alum) are favorable in point of enhancing the saline flow conductivity (SFC) more than that in a case of adding no water-soluble multivalent metal salts.

The liquid-permeability-enhancing agent is used in an amount of favorably 0.001 to 10 weight %, more favorably 0.01 to 5 weight %, relative to the water-absorbent resin particles.

Note that, the liquid-permeability-enhancing agent is not limited if it enhances liquid permeability, but preferably a substance which forms no covalent bonds with functional groups on the surface of the water-absorbent resin particles.

Examples of the another additive include: deodorizer, antimicrobial agent, fragrant material, blowing agent, pigment, dye, plasticizer, adhesive, surfactant, oxidizer, reducer, water, salts, chelating agent, bactericidal agent, hydrophilic polymer such as polyethyleneglycol, paraffin, hydrophobic polymer, thermoplastic resin such as polyethylene and polypropylene, and thermosetting resin such as polyester resin and urea resin. For example, the primary aqueous-liquid-absorbing agent according to the present invention may further include said other additive of the order of 0 to 10 weight %, relative to the water-absorbent resin particles.

Process for producing the primary aqueous-liquid-absorbing agent according to the present invention:

The process for producing a primary aqueous-liquid-absorbing agent herein includes, for example, at least the following steps:

(2.1) polymerizing a water-soluble ethylenically unsaturated monomer having a carboxyl group in an aqueous monomer solution including the water-soluble ethylenically unsaturated monomer having a carboxyl group, in the presence of an internal-crosslinking agent having at least four functional groups each capable of forming a covalent bond with a carboxyl group to thereby obtain a hydropolymer;

(2-3) drying the hydropolymer obtained in the step (2-1) at a temperature of not less than 150°C to thereby obtain water-absorbent resin particles; and

(2-5) surface-crosslinking the water-absorbent resin particles obtained in the step (2-3).

In addition, the process for a primary aqueous-liquid-absorbing agent according to the present invention may further include at least one of the following steps:

(2-2) before the step (2-1), further preparing an aqueous monomer solution including a water-soluble ethylenically unsaturated monomer having a carboxyl group and an internal-crosslinking agent having at least four functional groups each capable of forming a covalent bond with the carboxyl group;

(2-4) pulverizing the obtained hydropolymer or the obtained water-absorbent resin particles at least before or after the step (2-3);

(2-6) subjecting the water-absorbent resin particles to treatment for liquid permeability enhancement before or after the step (2-5).

The water-soluble ethylenically unsaturated monomer having a carboxyl group in an aqueous monomer solution including the water-soluble ethylenically unsaturated monomer having a carboxyl group, is polymerized in the presence of at least one internal-crosslinking agent to obtain a hydropolymer. As the internal-crosslinking agent, used is an internal-crosslinking agent having at least four functional groups each capable of forming a covalent bond with the carboxyl group of the water-soluble ethylenically unsaturated monomer. The water-soluble ethylenically unsaturated monomer having a carboxyl group is as described above. Other monomers may also be added in the polymerization process step.
Also, in this step, polymerization reaction is carried out in the presence of at least one inner-crosslinking agent. As the inner-crosslinking agent, at least an inner-crosslinking agent having at least four functional groups each of which forms a covalent bond with the carboxyl group of the water-soluble ethylenically unsaturated monomer is favorably used. This increases a saline flow conductivity (SFC) of the obtained water-absorbent resin particles, as compared with those obtained with a single use of (a) an internal-crosslinking agent having three or less functional groups each capable of forming a covalent bond with the carboxyl group of the water-soluble ethylenically unsaturated monomer or those obtained with the use of (b) an internal-crosslinking agent having at least two polymerizable ethylenical double bonds jointly with the internal-crosslinking agent (a).
The functional groups each capable of forming a covalent bond with a carboxyl group of the water-soluble ethylenically unsaturated monomer having the carboxyl group, are not particularly limited if they are functional groups which form bonds with the carboxyl group. Examples of the functional groups include: hydroxyl group, amino group, epoxy group, oxetane group, ethyleneimine group (aziridine group), isocyanate group, oxazoline, cyclocarbonate, cyclocarbonate, oxazolidinone, cyclic urea, azithidinium salt and chlorohydrin.
Therefore, examples of the internal-crosslinking agent having at least four functional groups each capable of forming a covalent bond with a carboxyl group include: an internal-crosslinking agent having at least four hydroxyl groups; an internal-crosslinking agent having at least four amino groups; an internal-crosslinking agent having at least four epoxy groups; an internal-crosslinking agent having at least four oxetane groups; an internal-crosslinking agent having at least four ethyleneimine groups (aziridine groups); an internal-crosslinking agent having at least four isocyanate groups; an internal-crosslinking agent having at least four oxazolines; an internal-crosslinking agent having at least four cyclocarbonates; an internal-crosslinking agent having at least four oxazolidinones; an internal-crosslinking agent having at least four cyclic ureas; an internal-crosslinking agent having at least four azithidinium salts; an internal-crosslinking agent having at least four chlorohydrins; and an internal-crosslinking agent having at least two kinds of functional groups selected from among hydroxyl group, amino group, epoxy group, oxetane group, ethyleneimine group (aziridine group), isocyanate group, oxazoline, cyclocarbonate, oxazolidinone, cyclic urea, azithidinium salt and chlorohydrin, wherein a combined total number of groups in the selected functional groups is at least four. Above all, the internal-crosslinking agent having at least four functional groups each capable of forming a covalent bond with a carboxyl group is more favorably the internal-crosslinking agent having at least four hydroxyl groups. Incidentally, in the case where the internal-crosslinking agent has a plurality of kinds of groups, a ratio between the groups is not particularly limited.
In addition, the internal-crosslinking agent having at least four functional groups each capable of forming a covalent bond with a carboxyl group may further have at least one polymerizable ethylenical double bond, ion-bondable functional group, or the like.
Moreover, the internal-crosslinking agent having at least four functional groups each capable of forming a covalent bond with a carboxyl group is not particularly limited if it has at least four functional groups each capable of forming a covalent bond with a carboxyl group. However, the number of the functional groups each capable of forming a covalent bond with a carboxyl group is favorably 4 to 50, more favorably 4 to 20, still more favorably 4 to 10, especially favorably 4 to 6. The number of carbon atoms of the internal-crosslinking agent is favorably 0.5 to 4 times as many as the number of the functional groups each capable of forming a covalent bond with a carboxyl group, more favorably 1 to 2 times. In the case where the number of such a functional groups is less than 4 or exceeds 50, the liquid permeability poorly enhances.

Specifically, examples of the internal-crosslinking agent having at least four hydroxyl groups include: polyhydric alcohol such as polyglycerol or pentaerythritol; sugar alcohol such as erythritol, xylitol, sorbitol, mannitol, maltitol, lactitol, or oligosaccharide alcohol; aldose such as xylose, glucose, gulose, mannose, or idose; and ketose such as fructose or sorbose. Examples of the internal-crosslinking agent having at least four amino groups include triethylenetetramine, tetraethylenepentamine, and pentaethylenehexamine. Examples of the internal-crosslinking agent having hydroxyl group and amino group include 2-amino-2-hydroxymethyl-1, 3-propanediol, and N,N-bis-(2-hydroxyethyl) ethylenediamine. These internal-crosslinking agents having at least four functional groups each capable of forming a covalent bond with a carboxyl group may be used either alone respectively or in combinations with each other.

Above all, the internal-crosslinking agent having at least four functional groups each capable of forming a covalent bond with a carboxyl group is more favorably the internal-crosslinking agent having at least four hydroxyl groups; more favorably sugar alcohol; still more favorably erythritol, xylitol, or sorbitol; especially favorably xylitol or sorbitol; most favorably sorbitol. These substances are favorably in view of their extremely high safety.

Further, the internal-crosslinking agent having at least four functional groups each capable of forming a covalent bond with a carboxyl group may be either high-molecular weight compound or low-molecular weight compound, but more favorably low-molecular weight compound. Its molecular weight is not particularly limited, but more favorably not more than 5000, more favorably not more than 2000, still more favorably not more than 1000, especially favorably not more than 500, most favorably not more than 200. Still further, a lower limit of the molecular weight of the internal-crosslinking agent having at least four functional groups each capable of forming a covalent bond with a carboxyl group is not particularly limited, but favorably 50, more favorably 80, still more favorably 90. The molecular weight of the above internal-crosslinking agent in the above range is favorably because internal crosslinking is carried out more efficiently. Further, in the case where polyvinyl alcohol, starch, or the like is used as an internal-crosslinking agent, there may occur coloring during drying. Therefore, the molecular weight is favorably in the above range.

Furthermore, the internal-crosslinking agent having at least four functional groups each capable of forming a covalent bond with a carboxyl group is favorably water-soluble such that it dissolves in amount of not less than 0.1 g in pure water of 100 g from the view points that it can be added easily and realize uniform crosslinking.

Additionally, other internal-crosslinking agents may be used. Examples of other internal-crosslinking agents, as described previously, includes: an internal-crosslinking agent having at least two polymerizable ethylenical double bonds and/or at least two functional groups per molecule. Specific examples of these other internal-crosslinking agents include: copolymerizable crosslinking agents having at least two polymerizable ethylenical double bonds, such as N,N'-methylenebis(meth)acrylamide, (poly)ethylene glycol di(meth)acrylate, (poly)propylene glycol di(meth)acrylate, trimethylolpropane tri(meth)acrylate, trimethylolpropane di(meth)acrylate, glycerol tri(meth)acrylate, glycerol acrylate methacrylate, ethylene-oxide-modified trimethylolpropane tri(meth)acrylate, pentaerythritol tetra(meth)acrylate, dipentaerythritol hexa(meth)acrylate, triallyl cyanurate, triallyl isocyanurate, triallyl phosphate, triallylamine, tetraallyloxyethane, pentaerythritol triallyl ether, and poly(meth)allyloxyalkanes. In addition, as examples of internal-crosslinking agents having a copolymerizable group having at least two polymerizable ethylenical double bonds and a covalent-bondable group, there can be cited such as (poly)ethylene glycol diglycidyl ether, glycerol diglycidyl ether, ethylenediamine, polyethylenimine, glycidyl (meth)acrylate, hydroxyethyl (meth)acrylate, hydroxypropyl (meth)acrylate, and glycidyl (meth)acrylate.

As to the amount of usage of this internal-crosslinking agent having at least four functional groups each of which forms a covalent bond with a carboxyl group, the amount of usage (Y) (unit: mol %) of the internal-crosslinking agent having at least four functional groups each capable of forming a covalent bond with a carboxyl group, relative to the water-soluble ethylenically unsaturated monomer, is favorably in the range expressed by the following equation (1):

$$Y \geq 0.06/\{2-(2.35X/100)\} \ \ldots(1).$$

In the equation (1) and the below-mentioned equation (2), X is a neutralization degree (unit: mol %) of a carboxyl group in the water-absorbent resin particles, and favorably in the range of 0.45 to 0.85. If Y falls outside the range expressed by the equation (1), internal crosslinking is carried out insufficiently, and thus a saline flow conductivity (SFC) of the obtained water-absorbent resin particles increases poorly. In addition, the water absorption capacity (CRC) exceeds 25g/g, and there is a possibility that a primary aqueous-liquid-absorbing agent having target performances of the present invention cannot be obtained.

The lower limit of the Y is, when it is Y = Z/{2-(2.35X/100)} ...(2), is a value such that it is favorably Z=0.06, more favorably Z=0.07, still more favorably Z=0.09, especially favorably Z=0.15.

The upper limit of the Y is a value such that it is favorably Z=1.2, more favorably Z=0.6, still more favorably Z=0.3 in the above equation (2). It is unfavorable that the Y is larger than 1.2/{2-(2.35X/100)}, because a water absorption capacity becomes too low.

In the case where the another internal-crosslinking agent is used, the total amount of the another internal-crosslinking agent as used is favorably in the range of 0 to 2 mol %, more favorably 0 to 1.5 mol %, still more favorably 0 to 1 mol %, particularly favorably 0 to 0.5 mol %, relative to the entire monomers (water-soluble ethylenically unsaturated monomers excluding the internal-crosslinking agents).

The above non-high-molecular weight compound and another internal-crosslinking agent may be added before polymerization of a monomer or added on the way of the polymerization, so as to exist at the time of the polymerization.

The polymerization reaction is preferably an aqueous solution polymerization in which the monomer is used in the form of an aqueous solution, optionally exceeding saturation. Such polymerization methods are disclosed in such as USP 4,625,001, USP 4,769,427, USP 4,873,299, USP 4,093,776, USP 4,367,323, USP 4,446,261, USP 4,683,274, USP 4,690,996, USP 4,721,647, USP 4,738,867, USP 4,748,076, and EP 1178059.

The temperature of the aqueous monomer solution is favorably in the range of 0 to 100 °C, more favorably 10 to 95 °C. (The saturated concentration is specified by the temperature of the aqueous monomer solution at normal pressures.) .

A polymer obtained in the aforementioned polymerization step is obtained as a hydropolymer. The obtained hydropolymer is, if necessary, pulverized in the form of a hydropolymer with water content of not less than 10 % but less than 70 %, for example. Further, the pulverized hydropolymer particles are dried. Conditions for drying the hydropolymer and pulverized hydropolymer particles are not particularly limited. However, the drying is carried out normally in the temperature range of 150 to 250 °C, favorably 150 to 220 °C, more favorably 160 to 200 °C, still more favorably 180 to 200 °C.

Drying at temperatures lower than 150 °C makes less prone to bringing an internal-crosslinking reaction. As a result of the drying, the hydropolymer or the pulverized hydropolymer particles obtained in such a manner that the hydropolymer is pulverized, if necessary, in the below-mentioned pulverizing step, favorably have a solid content in the range of 70 to 99.8 weight %, more favorably 80 to 99.7 weight %, still more favorably 90 to 99.5 weight %.

The process comprises typically a pulverizing step of pulverizing the obtained hydropolymer or water-absorbent resin particles before or after the drying step, favorably before and after the drying step. The resultant hydropolymer obtained in the polymerization step may be dried as it is. However, favorably, the resultant hydropolymer is pulverized before the drying step. For example, the resultant hydropolymer is extruded from a perforated structure having perforation diameters in the range of 0.3 to 18 mm to thus pulverize the hydropolymer to thereby form it into pulverized hydropolymer particles. By such extrusion of the hydropolymer from the perforated structure having the specific perforation diameters to thus pulverize the hydropolymer, it becomes possible to form it into the pulverized hydropolymer particles which can sufficiently exert the effects of the present invention. The shape of the perforations is such as a circular, quadrangular (e.g. square, rectangular), triangular, or hexagonal shape and is not especially limited. However, favorably, the hydropolymer is extruded from circular perforations. Incidentally, the aforementioned perforation diameters are defined as the diameters given in the case of converting the outer peripheries of the mesh opening portions into those of the circles.

The perforation diameters of the perforated structure for carrying out the extrusion pulverization in order to obtain the pulverized hydropolymer particles are more favorably in the range of 0.5 to 16 mm, still more favorably 0.5 to 12 mm, especially favorably 0.5 to 9.5 mm, most favorably 0.5 to 6.4 mm. In the case where the perforation diameters of the perforated structure are smaller than 0.3 mm, there is a possibility that the gel may become strings, or that the gel cannot be extruded. In the case where the perforation diameters of the perforated structure are larger than 18 mm, there is a possibility that the effects of the present invention cannot be exerted. Especially, there is a possibility that absorption rate (modified FSR) decreases.

Examples of the apparatus for carrying out the extrusion pulverization in order to obtain the pulverized hydropolymer particles include such as extrudes the hydropolymer from a perforated plate to thereby pulverize the hydropolymer. As the extrusion mechanism, there is used the mechanism of the type which can press-feed the hydropolymer from its supply inlet to the perforated plate, such as screw type or rotary roll type. The screw type extruder may be a single or multiple screw type and may be a type which is used usually for extrusion molding of edible meat, rubber, and plastic or used as a pulverizer. Examples thereof include meat choppers and Dome Gran.

It is favorable that at least a portion of the water-absorbent resin particles usable herein are agglomerate particles. More favorably, these agglomerate particles are those which are obtained by a process including the step of agglomeration of particles having particle diameters of smaller than 150 μm. The process for achieving such a mode that at least a portion of the water-absorbent resin particles are agglomerate particles is not especially limited and will do if hitherto publicly known agglomeration processes are applied thereto. Examples of such applicable processes include processes in which: warm water and a fine powder of water-absorbent resin particles are mixed together and then dried (USP 6,228,930); a fine powder of water-absorbent resin particles is mixed with an aqueous monomer solution, and then the resultant mixture is polymerized (USP 5,264,495); water is added to a fine powder of water-absorbent resin particles, and then the resultant mixture is agglomerated under not less than a specific face pressure (EP 0844270); a fine powder of water-absorbent resin particles is sufficiently wetted to thus form an amorphous gel, and then this gel is dried and pulverized (USP 4,950,692); and a fine powder of water-absorbent resin particles and a polymer gel are mixed together (USP 5,478,879).

In addition, it is favorable that at least a portion of the water-absorbent resin particles usable in the present invention are foamed particles. These foamed particles are favorably those which are obtained by a process characterized by including the step of polymerizing the monomer containing an azo initiator or a foaming agent (e.g. a carbonate) or polymerizing the monomer while it contains bubbles by causing it to bubble with an inert gas.

The agglomeration can be carried out at the same time as the gel pulverization if, as is aforementioned, the hydropolymer (obtained by polymerizing the aqueous monomer solution which has the specific monomer concentration and contains the specific internal-crosslinking agent in the specific amount) is extruded under the specific conditions (namely, extruded from the perforated structure having perforation diameters in the range of 0.3 to 18 mm) to thus pulverize the hydropolymer.

The water-absorbent resin particles or the primary aqueous-liquid-absorbing agent favorably has a bulk density in the range of 0.40 to 0.80 g/ml, more favorably 0.45 to 0.75 g/ml, still more favorably 0.50 to 0.70 g/ml.

The water-absorbent resin particles are subjected to surface-crosslinking. The surface-crosslinking step is carried out in at least one stage selected from among before, simultaneously with, and after the step of subjecting the water-absorbent resin particles to the later-described liquid permeability enhancing treatment step. However, the surface-crosslinking step is favorably carried out before the liquid permeability enhancing treatment step.

Examples of the surface-crosslinking agent usable for the surface-crosslinking treatment include: organic surface-crosslinking agents which have at least two functional groups reactable with a functional group (particularly, a carboxyl group) of the water-absorbent resin particles; multivalent metal compounds; and polycations. Examples thereof include: polyhydric alcohol compounds (e.g. ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerol, polyglycerol, 2-butene-1,4-diol, 1,3-butanediol, 1,4-butanediol, 1,5-pentanediol, 1,6-hexanediol, 1,2-cyclohexanedimethanol, 1,2-cyclohexanol, trimethylolpropane; diethanolamine, triethanolamine, polyoxypropylene, oxyethylene-oxypropylene block copolymers, pentaerythritol, and sorbitol); epoxy compounds (e.g. ethylene glycol diglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol); polyamine compounds (e.g. ethylenediamine, diethylenetriamine, triethylenetetramine, tetraethylenepentamine, pentaethylenehexamine, and polyethylenimine) and their inorganic or organic salts (e.g. azetidinium salts); polyisocyanate compounds (e.g. 2,4-tolylene diisocyanate and hexamethylene diisocyanate); polyoxazoline compounds (e.g. 1,2-ethylenebisoxazoline); carbonic acid derivatives (e.g. urea, thiourea, guanidine, dicyandiamide, 2-oxazolidinone); alkylene carbonate compounds (e.g. 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, 4,6-dimethyl-1,3-dioxan-2-one, and 1,3-dioxopan-2-one); haloepoxy compounds (e.g. epichlorohydrin, epibromohydrin, and $\alpha$-methylepichlorohydrin) and their polyamine-added products (e.g. Kymene (registered trademark) produced by Hercules); silane coupling agents (e.g. $\gamma$-glycidoxypropyltrimethoxysilane and $\gamma$-aminopropyltriethoxysilane); oxetane compounds (e.g. 3-methyl-3-oxetanemethanol, 3-ethyl-3-oxetanemethanol, 3-butyl-3-oxetanemethanol, 3-methyl-3-oxetaneethanol, 3-ethyl-3-oxetaneethanol, 3-butyl-3-oxetaneethanol, 3-chloromethyl-3-methyloxetane, 3-chloromethyl-3-ethyloxetane, and polyoxetane compounds); and multivalent metal compounds (e.g. hydroxides and chlorides of such as zinc, calcium, magnesium, aluminum, iron and zirconium). These surface-crosslinking agents may be used either alone respectively or in combinations with each other. Above all, it is more preferable to use a surface crosslinking agent which forms a covalent bond with a functional group (carboxyl group) on the surface of the water-absorbent resin particles because it can enhances the performance of absorbency against pressure. The polyhydric alcohols are favorable, because they are high in safety and can enhance the hydrophilicity of water-absorbent resin particle surfaces. In addition, the use of the polyhydric alcohols enhances the affinity of water-absorbent resin particle surfaces to the multivalent metal particles, so that interactions between the polyhydric alcohol residue and the multivalent metal surface enable more uniform existence of the multivalent metal particles on surfaces of the water-absorbent resin particles.

Although depending upon compounds as used, their combination, and others, the amount of the surface-crosslinking agent, as used, is favorably in the range of not less than 0.001 and not more than 10 weight parts, more favorably in the range of not less than 0.01 and not more than 5 weight parts, per 100 weight parts of the water-absorbent resin.

Although not especially limited, the method for mixing the water-absorbent resin particles and the surface-crosslinking agent together can be exemplified by such as: a method in which the water-absorbent resin particles are immersed into the hydrophilic organic solvent and then mixed with the surface-crosslinking agent that is, if necessary, dissolved in water and/or a hydrophilic organic solvent; and a method in which the surface-crosslinking agent that is dissolved in water and/or the hydrophilic organic solvent is spraywise or dropwise added directly to the water-absorbent resin particles to mix them together. In addition, in the case where the surface-crosslinking agent solution is sprayed, the size of liquid droplets being sprayed is favorably in the range of 1 to 300 μm, more favorably 2 to 200 μm.

After the mixing of the water-absorbent resin particles and the surface-crosslinking agent, usually, a heating treatment is favorably carried out to conduct the crosslinking reaction. Depending on the surface-crosslinking agent used, the temperature of the above heating treatment is favorably in the range of 40 to 250 °C, more favorably 150 to 250 °C. The duration of the heating treatment is favorably in the range of 1 minute to 2 hours, more favorably 5 minutes to 1 hour.

The process herein may include a liquid permeability enhancing treatment step for subjecting the water-absorbent resin particles to treatment for liquid permeability enhancement. The liquid permeability enhancing treatment step may be carried out at any time of before, simultaneously with, or after the surface-crosslinking step. However, it is favorable that the liquid permeability enhancing treatment step is carried out after the surface-crosslinking step and separately therefrom. The treatment for liquid permeability enhancement is not particularly limited if it is treatment for enhancing liquid permeability of the water-absorbent resin particles. However, the treatment for liquid permeability enhancement is favorably carried out by adding a liquid-permeability-enhancing agent, as described above

It may be dry-blend, or the liquid-permeability-enhancing agent may be added in the form of an aqueous solution, or the addition method may be carried out by heat-fusion. The heat-fusion is a method in which: the heating is carried out at the same time as or after mixing the multivalent metal hydrate (e.g. aluminum sulfate, potassium alum, ammonium alum, sodium alum) and the water-absorbent resin particles together; or the water-absorbent resin particles having been preheated are mixed with the multivalent metal compound; whereby the multivalent metal hydrate is fused and then made to adhere to the water-absorbent resin particles. If necessary, water may be added before the heating.

Test methods used herein:

Water absorption capacity (CRC: Centrifuge Retention Capacity)

An amount of 0.200 g of water-absorbent resin or (primary or secondary) aqueous-liquid-absorbing agent was uniformly placed into a bag (60 mm × 60 mm) made of nonwoven fabric (trade name: Heatron Paper, type: GSP-22, produced by Nangoku Pulp Kogyo Co., Ltd.) and then immersed into a physiological saline solution (hereinafter the physiological saline solution all refers to a 0.9 weight % aqueous sodium chloride solution) of which the temperature had been adjusted to 25 °C. After 30 minutes, the bag was pulled up and then drained of water by a centrifugal force of 250 G with a centrifugal separator (produced by Kokusan Co., Ltd., centrifugal separator: model H-122) for 3 minutes, and then the weight W1 (g) of the bag was measured. In addition, the same procedure as the above was carried out without the aqueous-liquid-absorbing agent, and the resultant weight W0 (g) was measured. Then, the CRC (g/g) was calculated from these W1 and W0 in accordance with the following equation:

$$\text{CRC (g/g)} = [(\text{W1 (g)} - \text{W0 (g)})/\text{weight (g) of water-absorbent resin}] - 1$$

Absorbency Against Pressure (AAP)

The absorbency against pressure (AAP) refers to absorbency against pressure for a physiological saline solution (0.9 weight % aqueous sodium chloride solution) under a load of 4.83 kPa in 60 minutes.

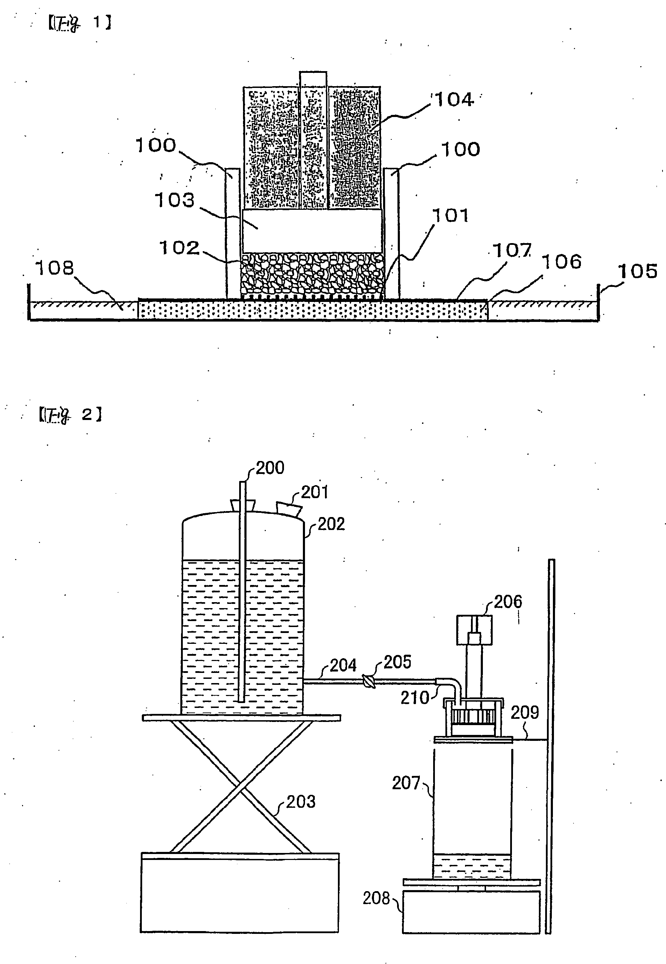

The measurement was carried out with an apparatus as shown in Fig. 1.

A stainless metal gauze 101, which was a screen of 400 meshes (mesh opening size: 38 μm), was fused to a bottom of a plastic supporting cylinder 100 having an inner diameter of 60 mm. Then, under conditions of a room temperature (23.0±2.0 °C) and a humidity of 50 RH%, onto the above metal gauze, there was uniformly spread 0.90 g of aqueous-liquid-absorbing agent 102, and further thereon, there were mounted a piston 103 and a load 104 in sequence, wherein the piston had an outer diameter of only a little smaller than 60 mm and made no gap with the inner wall surface of the supporting cylinder, but was not hindered from moving up and down, and wherein the piston and the load were adjusted so that a load of 4.83 kPa (0.7 psi) could uniformly be applied to the aqueous-liquid-absorbing agent. Then, the weight Wa (g) of the resultant one set of measurement apparatus was measured.

A glass filter plate 106 having a diameter of 90 mm (produced by Sogo Rikagaku Glass Seisakusho Co., Ltd., pore diameter: 100 to 120 μm) was mounted inside a Petri dish 105 having a diameter of 150 mm, and then a physiological saline solution (0.9 weight % aqueous sodium chloride solution) 108 (20 to 25 °C) was added up to the same level as the upside of the glass filter plate, on which a filter paper 107 having a diameter of 90 mm (produced by ADVANTEC Toyo Co., Ltd., trade name: (JIS P 3801, No.2), thickness: 0.26 mm, diameter of captured particles: 5 μm) was then mounted so that its entire surface would be wetted, and further, an excess of liquid was removed.

The one set of measurement apparatus was mounted on the above wet filter paper, thereby getting the liquid absorbed under the load for a predetermined duration. This absorption duration was defined as 1 hour from the start of the measurement. Specifically, 1 hour later, the one set of measurement apparatus was removed by being lifted to measure its weight Wb (g). This measurement of the weight must be carried out as quickly as possible and so as not to give any vibration. Then, the absorbency against pressure (AAP) (g/g) was calculated from the Wa and Wb in accordance with the following equation:

$$AAP \ (g/g) = [Wb \ (g) - Wa \ (g)]/\text{weight (g) of aqueous-liquid-absorbing agent}$$

Absorption rate (modified FSR: Free Swell Rate)

An amount (unit: g) (WA) (calculated from the below-mentioned equation (a)) of aqueous-liquid-absorbing agent was weighed out precisely to the fourth decimal place. This aqueous-liquid-absorbing agent as weighed out was placed into a 25 ml glass beaker (diameter: 32-34 mm, height: 50 mm), when the upside of the aqueous-liquid-absorbing agent as placed into the beaker was made horizontal. If necessary, a treatment such as of cautiously tapping the beaker may be carried out to make the surface of the aqueous absorbing agent horizontal. Next, 20 ml of physiological saline solution (0.9 weight % aqueous sodium chloride solution), of which the temperature had been adjusted to 23.0±2.0 °C, was weighed out into a 50 ml glass beaker, and then the weight (unit: g) was measured to the fourth decimal place (W1). Then, the physiological saline solution as weighed out was carefully and quickly poured into the 25 ml beaker containing the aqueous-liquid-absorbing agent. The time measurement was started at the same time as when the poured physiological saline solution contacted with the aqueous-liquid-absorbing agent. Then, the upside of the physiological saline solution in the beaker into which the physiological saline solution had been poured was observed at an angle of about 20° with the eye. Then, the time measurement was ended when the upside, which had been the liquid surface of the physiological saline solution at the start, had been replaced by the surface of the aqueous-liquid-absorbing agent (having absorbed the physiological saline solution) as a result of the absorption of the physiological saline solution into the aqueous-liquid-absorbing agent (unit: sec) (ts). Next, the weight (unit: g) of the physiological saline solution, which remained attaching to the 50 ml beaker after the pouring of the physiological saline solution, was measured to the fourth decimal place (W2). The weight (WF, unit: g) of the poured physiological saline solution was determined from the equation (b) below.

The water absorption rate (modified FSR) was calculated from the equation (c) below.

Equation (a):

$$WA \ (g) = 20 \ (g)/(0.75 \times CRC \ (g/g))$$

Equation (b):

$$WF \ (g) = W1 \ (g) - W2 \ (g)$$

Equation (c):

$$FSR \ (g/g/s) = WF/(ts \times WA)$$

The same measurement was carried out repeatedly three times per one sample. The measurement result was defined as the average value of the three-time-measured values.

Saline flow conductivity (SFC)

(SFC measurement apparatus)

This measurement is to measure the saline flow conductivity (SFC) of a gel layer formed in a (primary or secondary) aqueous-liquid-absorbing agent which has absorbed the physiological saline solution under load and thereby swollen.

This measurement of the saline flow conductivity (SFC) uses Darcy's law and the stationary-flow method (e.g. refer to "Absorbency", edited by P. K. Chatterjee, Elsevier 1985, pp. 42-43 and Chemical Engineering, Vol. II, 3rd edition, J. M. Coulson and J. F. Richarson, Pergamon Press, 1978, pp. 125-127).

An apparatus favorable for this measurement is illustrated in Fig. 2. This apparatus has a storage tank (202) of about 5 L in capacity as put on a laboratory jack (203). The storage tank (202) has an end-open glass tube and a rubber stopper part (200) which have been provided thereto in order to obtain a function of keeping the hydrostatic height constant. A liquid can be added to the storage tank (202) by removing a rubber stopper part (201). The storage tank (202) has a liquid outlet therein below the liquid surface, and a glass tube (204) having a valve (205) is connected to this outlet. The liquid feed can be controlled by opening and closing the valve (205). The glass tube (204) is connected to a flexible tube (210). The other end of the flexible tube (210) is set so as to feed a liquid to an SFC instrument (206) as illustrated in its entirety. The SFC instrument (206) was set on a support (209) having a stainless wire mesh of 1 mm in mesh opening size. Under the support (209), there is disposed a collection tank (207) for liquid collection. The collection tank (207) is disposed on a balance (208). The balance (208) is wired to a computer so that the mass of the collected liquid can be taken in every definite time.

Incidentally, in Fig. 2, in order to facilitate the understanding of this drawing figure, the right-hand apparatus (e.g. SFC instrument 206, collection tank 207, balance 208, support 209) is illustrated on a scale enlarged in comparison with the reduced scale of the left-hand apparatus.

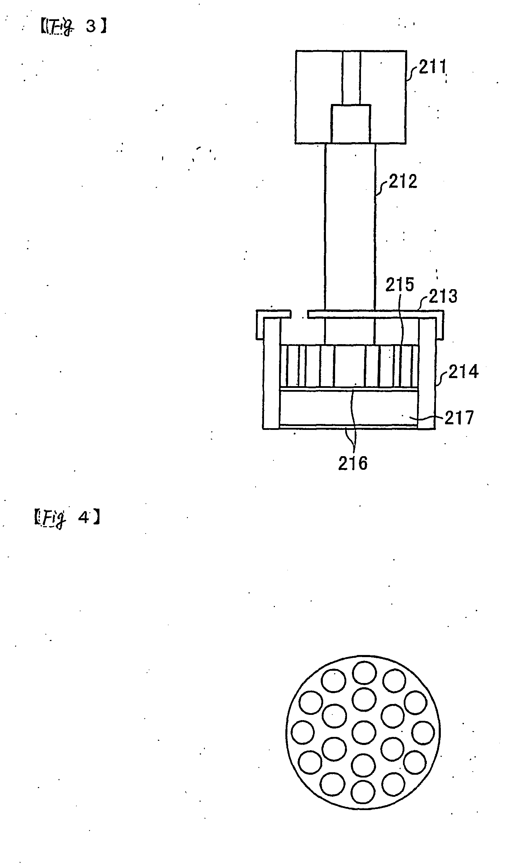

As to Fig. 3, the SFC instrument basically includes: a cylinder (214) (obtained by processing LEXANR or its equivalent) having a stainless wire mesh at the bottom; a piston (212) (obtained by processing LEXANR or its equivalent); a cover (213) (obtained by processing LEXANR or its equivalent) having an opening for insertion of the liquid-feeding tube; and a weight (211). The piston (212) has a piston head (215) through which holes are made as illustrated in Fig. 3. The holes of the piston head (215) have the cylindrical structure which is penetrating in upside and downside directions of the piston head (215) as illustrated in Fig. 4. On the bottom of the piston head (215), there is stuck a wire mesh (216) of 400 meshes (mesh opening size: 38 $\mu$m) (produced by Weisse & Eschrich, material: SUS 304, mesh opening width: 0.038 mm, wire diameter: 0.025 mm). The piston head (215) has a diameter only a little smaller than the inner diameter of the cylinder (214) and has such a size as allows the piston head (215) to smoothly migrate inside the cylinder (214) without being hindered from moving up and down. The top end of the shaft of the piston (212) is processed so that the weight can be set there. The cylinder (214) has an inner diameter of 6.00 cm (bottom area 28.27 cm2), a wall thickness of 0.5 cm, and a height of 6.0 cm. On the bottom of the cylinder (214), there is stuck a wire mesh (216) of 400 meshes (mesh opening size: 38 $\mu$m) (produced by Weisse & Eschrich, material: SUS 304, mesh opening width: 0.038 mm, wire diameter: 0.025 mm). The cover (213) has a hole of a size only a little larger than the external form of the shaft of the piston (212) so that the shaft of the piston (212) can smoothly migrate without being hindered from moving up and down. In addition, the cover (213) has the opening for insertion of the liquid-feeding tube. The total weight of the weight (211) and the piston (212) is adjusted so that a load of 2.07 kPa (0.3 psi) can be applied to the bottom of the cylinder.

(SFC measurement method)

First of all, the height (h0: unit = mm, number of significant figures = 4) and weight (W0: unit = g, number of significant figures = 4) of the SFC instrument, including the cylinder (214), the piston (212), the cover (213), and the weight (211), were measured before the aqueous-liquid-absorbing agent was placed into it, in other words, in an empty state.

Next, 3.00±0.05 g of aqueous-liquid-absorbing agent was weighed out (W: unit = g, number of significant figures = 4). The amount of the aqueous-liquid-absorbing agent being weighed out is favorably adjusted so that the below-mentioned "d final" will be in the range of 10 to 20 mm, more preferably 15 to 20 mm. For example, in the case where the water absorption capacity (CRC) is in the range of 5 to 16 g/g, the amount of the aqueous-liquid-absorbing agent being weighed out is adjusted to 3.00±0.05 g. In the case where the water absorption capacity (CRC) is in the range of above 16 to 20 g/g, the amount of the aqueous-liquid-absorbing agent being weighed out is adjusted to 2.00±0.03 g. In the case where the water absorption capacity (CRC) is in the range of above 20 to 25 g/g, the amount of the aqueous-liquid-absorbing agent being weighed out is adjusted to 1.60±0.03 g.

For a secondary aqueous liquid absorbing agent herein that has a water absorption capacity (CRC) above 25 g/g, the amount of the secondary aqueous liquid-absorbing agent being weight out is adjusted to 0.9 +/- 0.05g.

Note that the amount of the aqueous-liquid-absorbing agent being weighed out is favorably adjusted so that the below-mentioned "d final" will be in the above range. The weighed-out aqueous-liquid-absorbing agent was placed on the entire bottom of the cylinder (214) so as to carefully and uniformly be dispersed there. Thereafter, the piston (212), the cover (213), and the weight (211) are set to measure the height (h1: unit = mm) of the SFC instrument. Next, a physiological saline solution (0.9 weight % aqueous sodium chloride solution) was added into a Petri dish of at least 16 cm in diameter and at least 4 cm in height so that the SFC instrument could be immersed by at least 3 cm from its bottom. A filter paper of 90 mm in diameter (filter paper produced by ADVANTEC Co., Ltd.: No.2) was laid on the inner bottom of the Petri dish. The SFC instrument containing the aqueous-liquid-absorbing agent was mounted on the filter paper to swell the aqueous-liquid-absorbing agent for 60 minutes. After the aqueous-liquid-absorbing agent had been swollen for 60 minutes in this way, the SFC instrument was removed from the Petri dish to measure the height (h2: unit = mm, number of significant figures = 4) and weight (W2: unit = g, number of significant figures = 4) of the SFC instrument. Thereafter, the SFC instrument was moved and set onto the support (209) of the SFC measurement apparatus, and the flexible tube (210) was set into the insertion opening. Next, the valve (205) was opened to thereby start feeding the liquid. After this start of the liquid feeding, the hydrostatic height in the cylinder was adjusted so as to be kept at 5 cm until the amount (indicated by the balance) of the liquid, having passed through the gel layer and then been collected, reached about 200 g. This adjustment may be carried out either by adjusting the height of the laboratory jack (203) or by adjusting the height of the lower portion of the glass tube as inserted from the upper portion of the storage tank (202). When the hydrostatic height in the cylinder had been adjusted so as to be kept at 5 cm, then the weight data of the liquid, having passed through the gel layer and then been collected, started to be taken in by the computer as connected with the balance. The data intake was carried out every 5 sec until 180 sec. However, if the amount of the collected liquid reached not smaller than 2 kg within 180 sec after the start of the data intake, then, at that point of time (e.g. 120 sec), the data intake was ended. After the end of the data intake, the valve (205) was quickly closed. Thereafter, when the liquid had almost come not to flow down from the bottom of the cylinder (214) of the SFC instrument (when the hydrostatic height in the cylinder (214) had agreed with the height of the gel layer), the height (h3: unit = mm, number of significant figures = 4) of the SFC instrument was measured. Thereafter, the SFC instrument was moved onto a cylindrical instrument (having the same inner diameter as of the cylinder of the SFC instrument) to drip water off for 30 minutes. This operation is to put the SFC instrument on the cylindrical instrument to thereby make the dripping-off of water carried out favorably in a state where the bottom of the wire mesh, on which the aqueous-liquid-absorbing agent in the cylinder is disposed, is not in direct contact with anything. After the dripping-off of water had been carried out for 30 minutes in the above way, the height (h4: unit = mm, number of significant figures = 4) and weight (W4: unit = g, number of significant figures = 4) of the SFC instrument were measured.

(Calculation of SFC)

The data as taken in by the computer were plotted on a graph by indicating the time t (sec) as the X-axis and the weight (g) of the collected liquid as the Y-axis. The resultant plots were approximated to a straight line by the method of least squares, and then the rate (unit: g/s) of this straight line was determined.
The SFC was determined from the following equation:

$$\text{SFC (x } 10^{-7} \text{ cm}^3 \text{ s/g)}$$

$$= (\text{d final} \times \text{rate})/(\text{Area} \times \text{Density} \times \text{Pressure}) \times 10{,}000{,}000$$

wherein:

Area (cm2) = 28.27

Density (g/cm3) = 1.005 (the density of the 0.9 weight % physiological saline solution at 20 °C is used)

d final (cm) = {(h2 - h0) + (h3 - h0)}/2/10

Wet porosity

The measurement of the wet porosity is carried out subsequently to the measurement of the saline flow conductivity (SFC).

A five-ply filter paper (10 cm × 10 cm, produced by Ahlstrom, Grade: 989) was set on a horizontal experimental stand. Then, the SFC instrument having been subjected to the dripping-off of water for 30 minutes was put on the above five-ply filter paper for 10 minutes. Thereafter, the SFC instrument was moved onto a separately prepared new five-ply filter paper of the same as the aforementioned. After 16±2 hours, the height (h5: unit = mm) and weight (W5: unit = g) of the SFC instrument were measured. Incidentally, the specifications of the aforementioned filter papers are described in the EDANA strikethrough test.

The wet porosity was calculated from the following equation:

$$\text{Wet Porosity (unit: \%)} = [(W3 - W4 - 0.7)/\{h4 - h0\} \times 28.27]] \times 100$$

Particle diameters

Water-absorbent resin particles or aqueous-liquid-absorbing agents, having been pulverized, were classified with JIS standard sieves having mesh opening sizes of 850 μm, 710 μm, 600 μm, 500 μm, 425 μm, 300 μm, 212 μm, 150 μm, and 45 μm. Then, the percentages R of the residues on these sieves were plotted on a logarithmic probability paper. Therefrom, the weight-average particle diameter (D50) was read. Note that in the case where sizes of the water-absorbent resin particles or aqueous-liquid-absorbing agents exceed 850 μm, commercial JIS standard sieves having mesh opening sizes exceeding 850 μm are used appropriately.

Logarithmic standard deviation (σζ) of particle diameter distribution

Water-absorbent resin particles or aqueous-liquid-.absorbing agents were classified with JIS standard sieves having mesh opening sizes of 850 μm, 710 μm, 600 μm, 500 μm, 425 μm, 300 μm, 212 μm, 150 μm, and 45 μm. Then, the percentages R of the residues on these sieves were plotted on a logarithmic probability paper. Note that in the case where sizes of the water-absorbent resin particles or aqueous-liquid-absorbing agents exceed 850 μm, commercial JIS standard sieves having mesh opening sizes exceeding 850 μm are used appropriately. Thus, if X1 is defined as a particle diameter when R = 84.1 weight %, and if X2 is defined as a particle diameter when R = 15.9 weight %, then the logarithmic standard deviation (σζ) is shown by the following equation. The smaller σζ value shows the narrower particle diameter distribution.

$$\sigma\zeta = 0.5 \times \ln(X2/X1)$$

As to the classification method for measuring the particle diameters and the logarithmic standard deviation (σζ) of the particle diameter distribution, 10.0 g of water-absorbent resin particles or aqueous-liquid-absorbing agent was placed onto JIS standard sieves (having mesh opening sizes of 850 μm, 710 μm, 600 μm, 500 μm, 425 μm, 300 μm, 212 μm, 150 μm, and 45 μm) (THE IIDA TESTING SIEVE: diameter = 8 cm) and then classified with a shaking classifier (IIDA SIEVE SHAKER, TYPE: ES-65 type, SER. No. 0501) for 5 minutes.

Bulk density

The bulk density of the water-absorbent resin particles or aqueous-liquid-absorbing agent was measured by the method as described in Edana 460.1-99.

Basis Weight

The basis weight as used herein can be determined with the "European Disposables and Nonwovens Association" (EDANA) standard method for Mass per Unit Area (40.3-90).

Caliper

European Disposables and Nonwovens Association (EDANA) standard method for Thickness (No 30.5-99) is suitable used herein to determine the caliper. A suitable apparatus is described in paragraph 4.1. The specified pressure is 2.1 kPa.

Density of a region of the absorbent core or layer thereof

The density as used herein of a region or layer or core or article can be determined by divide the basis weight of that region of a layer or an absorbent core, determined using the Basis Weight method, by the caliper, determined by the Caliper method.

Extractable component content

Into a plastic receptacle of 250 ml in capacity having a lid, 184.3 g of physiological saline solution (0.9 weight % aqueous sodium chloride solution) was weighed out. Then, 1.00 g of water-absorbent resin or aqueous-liquid-absorbing agent was added to this aqueous solution, and they were stirred for 16 hours, thereby extractable components were extracted from the resin. The resultant extract liquid was filtrated with a filter paper (produced by ADVANTEC Toyo Co., Ltd., trade name: (JIS P 3801, No.2), thickness: 0.26 mm, diameter of captured particles: 5 $\mu$m), and then 50.0 g of the resultant filtrate was weighed out and used as a measuring solution.

To begin with, only the physiological saline solution was firstly titrated with an aqueous 0.1N NaOH solution until the pH reached 10, and then the resultant solution was titrated with an aqueous 0.1N HCl solution until the pH reached 2.7, thus obtaining blank titration amounts ([bNaOH] ml and [bHCl] ml).

The same titration procedure was carried out also for the measuring solution, thus obtaining titration amounts ([NaOH] ml and [HCl] ml).

For example, if the water-absorbent resin comprised acrylic acid and its sodium salt in known amounts, the extractable component content of the water-absorbent resin was calculated from the average molecular weight of the monomers and the titration amounts, as obtained from the above procedures, in accordance with the following equation. In the case of unknown amounts, the average molecular weight of the monomers was calculated from the neutralization degree as determined by the titration.

$$\text{Extractable component content (weight \%)} = 0.1 \times \text{(average molecular weight)} \times 184.3 \times 100 \times ([HCl] - [bHCl])/1,000/1.0/50.0$$

$$\text{Neutralization degree (mol \%)} = [1 - ([NaOH] - [bNaOH])/([HCl] - [bHCl])] \times 100$$

Example 1

In a reactor as formed by lidding a jacketed stainless twin-arm kneader of 10 liters in capacity having two sigma-type blades, there was prepared a reaction liquid by dissolving 52.63 g (0.4 mol %) of polyethylene glycol diacrylate and 18.33 g (0.4 mol %) of D-sorbitol into 5,367.3 g of aqueous solution of sodium acrylate having a neutralization degree of 60 mol % (monomer concentration: about 40 weight %). Next, this reaction liquid was deaerated under an atmosphere of nitrogen gas for 20 minutes. Subsequently, 30.19 g of 10 weight % aqueous sodium persulfate solution and 25.16 g of 0.1 weight % aqueous L-ascorbic acid solution were added thereto under stirred conditions. As a result, polymerization started after about 1 minute. Then, the polymerization was carried out in the range of 20 to 95 °C while the forming gel was pulverized. Then, the resultant hydropolymer was got out after 30 minutes from the start of the polymerization. The resultant hydropolymer was in the form of divided small pieces having diameters of not larger than about 5 mm.

The resultant hydropolymer was pulverized and agglomerated with a screw extruder (produced by Hiraga Kosakusho, Chopper, MODEL: TB-32, perforation diameter of perforated plate = 9.5 mm, thickness of perforated plate = 5.0 mm, number of revolutions of screw = 32.5 rpm), thus obtaining pulverized hydropolymer particles having been divided into small pieces. Incidentally, the hydropolymer was supplied at 1300 g/minute.

The resultant pulverized hydropolymer particles having been divided into small pieces were spread onto a metal gauze of 50 meshes (opening size: 300 $\mu$m) and then dried with hot air of 180 °C for 40 minutes. Next, the dried product was pulverized with a roll mill and then classified with JIS standard sieves having a mesh opening size of 600 $\mu$m and 150 $\mu$m, thus obtaining a water-absorbent resin (having a solid content of 96 weight parts) of an irregularly pulverized shape, which had a weight-average particle diameter of 324 $\mu$m and a logarithmic standard deviation ($\sigma\zeta$) of 0.32.

An amount of 500 weight parts of the obtained water-absorbent resin was placed into Lödige Mixer (produced by Lödige, type: M5R) and then uniformly spraywise mixed with a surface-crosslinking agent solution comprising a mixed liquid of 2.4 weight parts of 1,4-butanediol, 3.8 weight parts of propylene glycol, and 20.0 weight parts of pure water under stirring. Then, the water-absorbent resin, which had been mixed with the surface-crosslinking agent solution, was placed into a stainless reactor (diameter: about 30 cm, height: about 20 cm) having a stirrer. Then, the reactor was immersed into an oil bath (of which the temperature had been adjusted to 200 °C) to carry out heat treatment for surface-crosslinking under stirring for 35 minutes. After this heat treatment, the resultant water-absorbent resin was disintegrated to such a degree that it could pass through a JIS standard sieve having a mesh opening size of 600 $\mu$m. As a result, surface-crosslinked water-absorbent resin particles were obtained.

An amount of 100 weight parts of the resultant surface-crosslinked water-absorbent resin particles was heated to 150 °C and then uniformly mixed with 1.6 weight parts of potassium alum (potassium aluminum sulfate dodecahydrate) under stirring for 5 minutes, thus obtaining a primary aqueous-liquid-absorbing agent (1).

Example 2

A primary aqueous-liquid-absorbing agent (2) was obtained in the same way as of Example 1 except that: the perforation diameter of perforated plate in Example 1 was changed to 6.5 min. The physical properties of the primary aqueous-liquid-absorbing agent (2) are shown in Table 1. Incidentally, the resultant primary aqueous-liquid-absorbing agent (2) had an extractable component content of 1.1 weight %.

Comparative Example 1

| Examples | Aqueous-liquid-absorbing agents | SFC ($\times$ 10$^{-7}$ cm$^3$ s/g) | CRC (g/g) | FSR (g/g/s) | Wet Porosity (%) | D50 ($\mu$m) | Ratio of 150-600 $\mu$m (wt%) |
|---|---|---|---|---|---|---|---|
| 1 | (1) | 1564 | 9.1 | 0.18 | 41.3 | 353 | 98.3 |
| 2 | (2) | 1632 | 9.2 | 0.23 | 41.9 | 321 | 98.4 |

A comparative primary aqueous-liquid-absorbing agent (1) was obtained in the same way as of Example 1 except that: 52.63 g (0.4 mol %) of polyethylene glycol diacrylate and 18.33 g (0.4 mol %) of D-sorbitol were replaced with 13.16 g (0.1 mol %) of polyethylene glycol diacrylate and 13.90 g (0.6 mol %) of glycerine; the perforation diameter of perforated plate in the screw extruder was changed to 4.5 mm; and the number of revolutions of screw was changed to 32.5 rpm.

Comparative examples

A comparative primary aqueous-liquid-absorbing agent (2) was obtained in the same way as of Example 1 except that: 52.63 g (0.4 mol %) of polyethylene glycol diacrylate and 18.33 g (0.4 mol %) ofD-sorbitol were replaced with 92.11 g (0.7 mol %) of polyethylene glycol diacrylate alone; the perforation diameter of perforated plate in the screw extruder was changed to 4.5 mm; the number of revolutions of screw was changed to 32.5 rpm; a mixture liquid of 2.4 weight parts of 1,4-butanediol, 3.8 weight parts of propylene glycol, and 20 weight parts of pure water is replaced with a mixture liquid of 2.3 weight parts of 1,4-butanediol, 4.5 weight parts of propylene glycol, and 22.5 weight parts of pure water.

Table 2:

| Comparative Examples | Comparative aqueous-liquid-absorbing agents | SFC (x10$^{-7}$ cm$^3$ s/g) | CRC (g/g) | FSR (g/g/s) | Wet Porosity (%) | D50 ($\mu$m) | Ratio of 150-600 $\mu$m (wt%) |
|---|---|---|---|---|---|---|---|
| 1 | (1) | 953 | 10.1 | 0.24 | 37.3 | 324 | 97.5 |
| 2 | (2) | 1000 | 12.1 | 0.34 | 39.0 | 350 | 99.9 |

**Claims**

1. An absorbent article comprising an absorbent core, which comprises a primary aqueous-liquid-absorbing agent that contains water-absorbent resin particles, said resin particles being internally cross-linked and surface-crosslinked, whereby the primary aqueous-liquid-absorbing agent exhibits a water absorption capacity (CRC) of 5 to 25 g/g and a saline flow conductivity (SFC) of not less than 1216 x $10^{-7}$ $cm^3$ s/g, and whereby said primary aqueous-liquid-absorbing agent is obtainable by a process comprising the step a) of:

polymerizing a water-soluble ethylenically unsaturated monomer having a carboxyl group in an aqueous monomer solution including the water-soluble ethylenically unsaturated monomer having a carboxyl group, in the presence of an internal-crosslinking agent having at least four functional groups each capable of forming a covalent bond with a carboxyl group.

2. An absorbent article as in claim1, whereby in said process step a) a hydropolymer is obtained; and whereby said process comprises the steps of:

(b) drying the hydropolymer obtained in the step (a) at a temperature of not less than 150°C to thereby obtain water-absorbent resin particles; and
(c) surface-crosslinking the water-absorbent resin particles obtained in the step (b),
wherein the amount (Y) (mol %) of the internal-crosslinking agent as used, relative to the water-soluble ethylenically unsaturated monomer having a carboxyl group, is expressed by the following equation (1):

$$Y \geq 0.06/\{2-(2.35X/100)\} \quad ...(1)$$

where X is neutralization degree (mol %) of a carboxyl group in the water-absorbent resin particles and is in the range of 45 to 85 mol %.

3. A disposable absorbent article as in claim 1 or 2 whereby the absorption rate (modified FSR) of the primary aqueous-liquid-absorbing agent is not less than 0.1 g/g/s.

4. A disposable absorbent article as in any of claim 1 to 3, whereby the primary aqueous-liquid-absorbing agent has a wet porosity of not less than 20 %.

5. A disposable absorbent article as in any preceding claim whereby the water-absorbing resin particles comprise agglomerated particles and the primary aqueous-liquid-absorbing agent is particulate, having at least 90 weight % by weight of particles with a particle diameters in the range of 150 to 850 $\mu$m.

6. A disposable absorbent article as in any preceding claim whereby said primary aqueous-liquid-absorbing agent further comprises a liquid-permeability-enhancing agent, selected from water-soluble metal compounds and water-soluble polycationic compounds.

7. A disposable absorbent article as in any preceding claim comprising a secondary aqueous-liquid-absorbing agent, comprising water-absorbing resin particles, having a SFC of less than 600 x $10^{-7}$ $cm^3$ s/g, preferably a SFC of between 40 x$10^{-7}$ $cm^3$ s/g and 400x$10^{-7}$$cm^3$ s/g.

8. A disposable absorbent article as in claim 7, comprising an absorbent core comprising an acquisition/ storage layer and a storage layer, whereby said primary aqueous-liquid-absorbing agent is comprised in at least part of said acquisition/ storage layer and whereby said secondary aqueous-liquid-absorbing agent is comprised in at least part of said storage region.

9. A disposable absorbent article as in any preceding claim, comprising an absorbent core that comprises a layer comprising said primary aqueous-liquid-absorbing agent and less than 10% by weight of said agent of a fibrous cellulose material.

10. A disposable absorbent article comprising as in any preceding claim, having a maximum dry caliper in the crotch region (as measured herein) of 4.5 mm or less.

11. A disposable absorbent article as in any preceding claim, which comprises an absorbent core comprising at least said primary aqueous-liquid-absorbing agent and optionally said secondary aqueous-liquid-absorbing agent, and said core having one or more regions with an average density greater than about 0.2 $g/cm^3$

**Patentansprüche**

1. Absorptionsartikel, der einen Absorptionskern umfasst, der ein primäres Wasser/Flüssigkeits-Absorptionsmittel umfasst, das wasserabsorbierende Harzpartikel enthält, wobei die Harzpartikel intern vernetzt und oberflächenvernetzt sind, wobei das primäre Wasser/Flüssigkeits-Absorptionsmittel ein Wasserabsorptionsvermögen (CRC) von 5 bis 25 g/g und eine Salzlösungsfluss-Leitfähigkeit (SFC) von nicht weniger als 1216 x $10^{-7}$cm$^3$ s/g aufweist, und wobei das primäre Wasser/Flüssigkeits-Absorptionsmittel anhand eines Verfahrens erhalten werden kann, dass folgenden Schritt a) umfasst:

   Polymerisieren eines wasserlöslichen, ethylenisch ungesättigten Monomers, das eine Carboxylgruppe, in einer wässrigen Monomerlösung aufweist, welche das wasserlösliche, ethylenisch ungesättigte Monomer, das eine Carboxylgruppe aufweist, enthält, in Anwesenheit eines internen Vernetzungsmittels mit mindestens vier funktionalen Gruppen, von denen jede in der Lage ist, eine kovalente Bindung mit einer Carboxylgruppe zu bilden.

2. Absorptionsartikel nach Anspruch 1, wobei in dem Verfahrensschritt a) ein Hydropolymer erhalten wird; und wobei das Verfahren die folgenden Schritte umfasst:

   (b) Trocknen des in Schritt a) erhaltenen Hydropolymers bei einer Temperatur von nicht weniger als 150 °C, um **dadurch** wasseradsorbierende Harzteilchen zu erhalten; und

   (c) oberflächliches Vernetzen der wasserabsorbierenden Harzteilchen, die in Schritt (b) erhalten wurden, wobei die Menge (Y) (Mol-%) an internem Vernetzungsmittel, wie verwendet, in Bezug auf das wasserlösliche, ethylenisch ungesättigte Monomer, das eine Carboxylgruppe aufweist, durch die folgende Gleichung (1) ausgedrückt wird:

$$Y \geq 0,06/\{2-(2,35X/100)\} \ \ldots(1)$$

   wobei X ein Neutralisationsgrad (Mol-%) einer Carboxylgruppe in den wasserabsorbierenden Harzteilchen ist und im Bereich von 45 bis 85 Mol-% liegt.

3. Wegwerf-Absorptionsartikel nach Anspruch 1 oder 2, wobei die Absorptionsrate (modifizierte FSR) des primären Wasser/Flüssigkeits-Absorptionsmittels nicht unter 0,1 g/g/s liegt.

4. Wegwerf-Absorptionsartikel nach einem der Ansprüche 1 bis 3, wobei das primäre Wasser/Flüssigkeits-Absorptionsmittel eine Nassporosität von nicht weniger als 20 % aufweist.

5. Wegwerf-Absorptionsartikel nach einem der vorstehenden Ansprüche, wobei die wasserabsorbierenden Harzteilchen agglomerierte Teilchen aufweisen und das primäre Wasser/Flüssigkeits-Absorptionsmittel in Teilchenform vorliegt und mindestens 90 Gew.-% Teilchen mit einem Teilchendurchmesser im Bereich von 150 bis 850 μm aufweist.

6. Wegwerf-Absorptionsartikel nach einem der vorstehenden Ansprüche, wobei das primäre Wasser/Flüssigkeits-Absorptionsmittel ferner ein die Flüssigkeitspermeabilität verbesserndes Mittel umfasst, das ausgewählt ist aus wasserlöslichen Metallverbindungen und wasserlöslichen polykationischen Verbindungen.

7. Wegwerf-Absorptionsartikel nach einem der vorstehenden Ansprüche, das ein sekundäres Wasser/Flüssigkeits-Absorptionsmittel umfasst, das wasserabsorbierende Harzteilchen mit einer SFC von weniger als 600 x $10^{-7}$cm$^3$ s/g, vorzugsweise einer SFC zwischen 40 x $10^{-7}$cm$^3$ s/g und 400 x $10^{-7}$cm$^3$ s/g umfasst.

8. Wegwerf-Absorptionsartikel nach Anspruch 7, das einen Absorptionskern umfasst, der eine Aufnahme/Speicherschicht und eine Speicherschicht umfasst, wobei das primäre Wasser/Flüssigkeits-Absorptionsmittel in zumindest einem Teil der Aufnahme/Speicherschicht enthalten ist, und wobei das sekundäre Wasser/Flüssigkeits-Absorptionsmittel in zumindest einem Teil der Speicherregion enthalten ist.

9. Wegwerf-Absorptionsartikel nach einem der vorstehenden Ansprüche, der einen Absorptionskern umfasst, der eine Schicht umfasst, die das primäre Wasser/Flüssigkeits-Absorptionsmittel und zu mindestens 10 Gew.-% das Mittel aus faserartigem Cellulosematerial umfasst.

**10.** Wegwerf-Absorptionsartikel nach einem der vorstehenden Ansprüche mit einer maximalen Trockendicke in der Schrittregion (wie hier gemessen) von 4,5 mm oder weniger.

**11.** Wegwerf-Absorptionsartikel nach einem der vorstehenden Ansprüche, der einen Absorptionskern umfasst, der mindestens das primäre Wasser/Flüssigkeits-Absorptionsmittel und optional das sekundäre Wasser/Flüssigkeits-Absorptionsmittel umfasst, und wobei der Kern eine oder mehrere Regionen mit einer durchschnittlichen Dichte von mehr als etwa 0,2 g/cm$^3$ aufweist.

## Revendications

**1.** Article absorbant comprenant une âme absorbante, qui comprend un agent d'absorption des liquides aqueux primaire qui contient des particules de résine absorbant l'eau, lesdites particules de résine réticulées de façon interne et réticulées en surface, selon lequel l'agent d'absorption des liquides aqueux primaire présente une capacité d'absorption d'eau (CAE) de 5 à 25 g/g et une conductivité en flux salin (CFS) de pas moins de 1216 x 10$^{-7}$cm$^3$ s/g, et selon lequel ledit agent d'absorption des liquides aqueux primaire peut être obtenu par un procédé comprenant l'étape a) consistant à :

polymériser un monomère éthyléniquement insaturé hydrosoluble ayant un groupe carboxyle dans une solution de monomère aqueuse incluant le monomère éthyléniquement insaturé hydrosoluble ayant un groupe carboxyle, en présence d'un agent de réticulation interne ayant au moins quatre groupes fonctionnels susceptibles chacun de former une liaison covalente avec un groupe carboxyle.

**2.** Article absorbant selon la revendication 1, selon lequel dans ladite étape de procédé a) un hydropolymère est obtenu ; et selon lequel ledit procédé comprend les étapes consistant à :

(b) sécher l'hydropolymère obtenu dans l'étape (a) à une température de pas moins de 150 °C pour obtenir de ce fait des particules de résine absorbant l'eau ; et
(c) réticuler en surface les particules de résine absorbant l'eau obtenues dans l'étape (b),
dans lequel la quantité (Y) (% molaire) de l'agent de réticulation interne tel qu'il est utilisé, par rapport au monomère éthyléniquement insaturé hydrosoluble ayant un groupe carboxyle, est exprimée par l'équation suivante (1) :

$$Y \geq 0,06/\{2-(2,35X/100)\} \ \ldots(1)$$

où X est le degré de neutralisation (% molaire) d'un groupe carboxyle dans les particules de résine absorbant l'eau et est dans l'intervalle de 45 à 85 % molaire.

**3.** Article absorbant jetable selon la revendication 1 ou 2, selon lequel le taux d'absorption (TGR modifié) de l'agent d'absorption des liquides aqueux primaire n'est pas inférieur à 0,1 g/g/s.

**4.** Article absorbant jetable selon l'une quelconque des revendications 1 à 3, selon lequel l'agent d'absorption des liquides aqueux primaire a une porosité mouillée de pas moins de 20 %.

**5.** Article absorbant jetable selon l'une quelconque des revendications précédentes, selon lequel les particules de résine absorbant l'eau comprennent des particules agglomérées et l'agent d'absorption des liquides aqueux primaire est particulaire, ayant au moins 90 % en poids de particules avec des diamètres de particule dans l'intervalle de 150 à 850 μm.

**6.** Article absorbant jetable selon l'une quelconque des revendications précédentes, selon lequel ledit agent d'absorption des liquides aqueux primaire comprend en outre un agent renforçant la perméabilité aux liquides, choisi parmi des composés métalliques hydrosolubles et des composés polycationiques hydrosolubles.

**7.** Article absorbant jetable selon l'une quelconque des revendications précédentes comprenant un agent d'absorption des liquides aqueux secondaire, comprenant des particules de résine absorbant l'eau, ayant une CFS de moins de 600 x 10$^{-7}$ cm$^3$ s/g, de préférence une CFS comprise entre 40 x 10$^{-7}$ cm$^3$ s/g et 400 x 10$^{-7}$ cm$^3$ s/g.

8. Article absorbant jetable selon la revendication 7, comprenant une âme absorbante comprenant une couche de recueil / stockage et une couche de stockage, selon lequel ledit agent d'absorption des liquides aqueux primaire est compris dans au moins une partie de ladite couche de recueil / stockage et selon lequel ledit agent d'absorption des liquides aqueux secondaire est compris dans au moins une partie de ladite région de stockage.

9. Article absorbant jetable selon l'une quelconque des revendications précédentes, comprenant une âme absorbante qui comprend une couche comprenant ledit agent d'absorption des liquides aqueux primaire et moins de 10 % en poids dudit agent d'un matériau à base de cellulose fibreux.

10. Article absorbant jetable selon l'une quelconque des revendications précédentes, ayant une épaisseur à l'état sec maximale dans la région d'entrejambe (telle que mesurée ici) de 4,5 mm ou moins.

11. Article absorbant jetable selon l'une quelconque des revendications précédentes, qui comprend une âme absorbante comprenant au moins ledit agent d'absorption des liquides aqueux primaire et facultativement ledit agent d'absorption des liquides aqueux secondaire, et ladite âme ayant une ou plusieurs régions avec une masse volumique moyenne supérieure à environ 0,2 g/cm$^3$.

【Fig 1】

【Fig 2】

【Fig 3】

211
212
215
213
214
217
216

【Fig 4】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0504491 A
- WO 05097313 A
- US 4940464 A
- US 5554145 A
- US 5569234 A
- US 6004306 A
- US 171249 A
- US 824121 A
- US 3860003 A
- US 5151092 A
- US 4610678 A
- US 4834735 A
- US 5260345 A
- US 5387207 A
- US 5397316 A
- US 5625222 A
- US 776839 A
- US P4625001 A
- US P4654039 A
- US P5250640 A
- US P5275773 A
- EP 0456136 A

- US P4076663 A
- US P4389513 A
- US P4124748 A
- US P3959569 A
- US P3935099 A
- US P4769427 A
- US P4873299 A
- US P4093776 A
- US P4367323 A
- US P4446261 A
- US P4683274 A
- US P4690996 A
- US P4721647 A
- US P4738867 A
- US P4748076 A
- EP 1178059 A
- US P6228930 B
- US P5264495 A
- EP 0844270 A
- US P4950692 A
- US P5478879 A

**Non-patent literature cited in the description**

- Absorbency. Elsevier, 1985, 42-43

- **J. M. Coulson ; J. F. Richarson.** Chemical Engineering. Pergamon Press, 1978, vol. II, 125-127